# EUROPEAN PATENT APPLICATION

(11) **EP 4 501 953 A1**
(43) Date of publication of application: **05.02.2025**
(21) Application number: 23777635.6
(22) Date of filing: 07.02.2023
(51) Int. Cl.: C07K 14/705, C07K 16/28, C12N 5/10, C12N 15/62, C12N 15/85, A61P 35/00, A61K 35/17, A61K 48/00

(54) **ANTIBODY THAT SPECIFICALLY BINDS TO CD7 AND USE THEREOF IN PREPARING CHIMERIC ANTIGEN RECEPTOR**

(30) Priority: 29.03.2022 CN 202210317175; 18.05.2022 CN 202210542681
(71) Applicant: Institute of Hematology and Blood Diseases Hospital, Chinese Academy of Medical Sciences, Tianjin 300020 (CN)
(72) Inventor: WANG, Jianxiang, Tianjin 300020 (CN); WANG, Min, Tianjin 300020 (CN); XIE, Leling, Tianjin 300020 (CN); GU, Runxia, Tianjin 300020 (CN); YANG, Xue, Tianjin 300020 (CN); RAO, Qing, Tianjin 300020 (CN); LIAO, Xiaolong, Tianjin 300020 (CN)
(74) Representative: Cabinet Chaillot
(86) International application number: PCT/CN2023/074721
(87) International publication number: WO 2023/185256

(57) **Abstract**

Provided are an antibody that specifically binds to CD7 and an antigen-binding moiety thereof. The antibody and the antigen-binding moiety thereof have high affinity and high binding specificity for CD7. Also provided are a CD7-targeting chimeric antigen receptor CD7 scFv-CD8α-4-1BB-CD3ζ, and a T cell that expresses the chimeric antigen receptor. The T cell can effectively prevent off-target effects.

## Description

### CROSS REFERENCE TO RELATED APPLICATIONS

The present application claims priority to Chinese Patent Application No. 202210317175.X earliest submitted to China National Intellectual Property Administration on March 29, 2022 and entitled "ANTIBODY THAT SPECIFICALLY BINDS TO CD7 AND USE THEREOF IN PREPARING CHIMERIC ANTIGEN RECEPTOR" and Chinese Patent Application No. 202210542681.9 submitted to China National Intellectual Property Administration on May 18, 2022 and entitled "ANTIBODY THAT SPECIFICALLY BINDS TO CD7 AND USE THEREOF IN PREPARING CHIMERIC ANTIGEN RECEPTOR", which are hereby incorporated by reference in their entirety.

### BACKGROUND OF THE INVENTION

### 1. Technical Field

The present invention relates to the field of biomedicine, and in particular, to an antibody specifically binding to CD7 and a use thereof in preparing a chimeric antigen receptor.

### 2. Description of Related Art

Human CD7 molecules are a member of the immunoglobulin superfamily and are expressed on surfaces of thymocytes, NK cells, and over 90% of T cells. At present, CD7 coupled immunotoxins and CD7 nano-antibodies have been developed to target leukemia/lymphoma tumor cells or human T cells for tumor clearance or prevention/treatment of graft-versus-host diseases. In addition, studies show that specific siRNA is delivered to T cells by coupling CD7 molecules with corresponding proteins, so as to control and treat human immunodeficiency diseases (HIVs). Although CD7 molecules are not essential for cell survival, the specific functions performed by CD7 molecules in T/NK cells are still unknown, and only a few early studies suggest that CD7 molecules may interact with TCR activation pathways to affect the activation of T cells. A great deal of research and exploration are needed to interpret the physiological/pathological functions of CD7 molecules in immune cells/tumor cells.

In recent years, the incidence of patients with acute T lymphoblastic leukemia (T-ALL) has increased gradually. The overall 5-year survival rate of patients is only about 50%. The cure rate of childhood patients is slightly higher than that of adult patients, while the long-term survival rate of adult relapsed and refractory patients is less than 10%. For relapsed and refractory patients, the application effect of new drugs is limited. Hematopoietic stem cell transplantation often becomes the only treatment choice, but its application is limited by many factors such as patient's disease status, bone marrow matching results, transplant related toxicity, and side effects. Therefore, such patients urgently need effective novel therapeutic drugs to alleviate their disease burden and achieve the therapeutic goals of disease relief or bridging bone marrow transplantation.

Antigens such as CD2, CD5, and CD7 are often highly expressed in tumor cells of T-ALL patients, among which CD7 antigen is generally highly expressed to provide therapeutic targets for antibody therapy and chimeric antigen receptor T cell (CAR-T) immunotherapy. However, patient's tumor cells are abnormal T cells, and the high tumor load in the patient's body inhibits the proliferation of normal T cells, so it is difficult to collect autologous T cells from patients to prepare CAR-T in clinical treatment, and the potential problem of mixed tumor cells is one of the safety hazards of treatment. Therefore, constructing a universal CAR-T product from allogeneic sources for the treatment of T-ALL is particularly crucial. On the other hand, because normal T cells also express CD7 antigen (with a positive expression rate of over 90%), in order to achieve effective proliferation of CAR-T cells, "cells kill each other" need to be avoided. Existing technologies still need to be explored and improved to solve the above problems, and antibodies and chimeric antigen receptors with better effects need to be further researched and developed. Therefore, searching for the antibodies and chimeric antigen receptors has become an urgent task.

### BRIEF SUMMARY OF THE INVENTION

One aspect of the present invention is to provide an antibody specifically binding to CD7 and a use thereof in preparing a chimeric antigen receptor in response to the problem of poor affinity of antibodies specifically binding to CD7 in existing technologies.

A technical solution provided by the present invention is as follows:

An antibody specifically binding to CD7 includes CDR1, CDR2, and CDR3 sequences of a light chain variable region as shown in SEQ ID No. 1-3 and CDR1, CDR2, and CDR3 sequences of a heavy chain variable region as shown in SEQ ID No. 4-6, or sequences having more than 80% of homology to the sequences.

In some implementations of the present invention, point mutations may be performed on the complementary determining regions (CDRs), with the aim of, for example, obtaining better affinity and/or dissociation property, obtaining better expression level, etc., as long as they can form complementary binding structures with antigenic determinants. The mutated amino acid sequences are considered to fall within the scope of protection of the present invention.

Another aspect of the present invention is to provide an antigen-binding moiety specifically binding to CD7. The antigen-binding moiety includes CDR1, CDR2, and CDR3 sequences of a light chain variable region as shown in SEQ ID No. 1-3 and CDR1, CDR2, and CDR3 sequences of a heavy chain variable region as shown in SEQ ID No. 4-6, or sequences having more than 80% of homology to the sequences.

In the present invention, the more than 90% of homology refers to more than 90% of homology to the nucleotide and amino acid sequences described in the present invention, and may be 80%, 81%, 82%, 83%, 84%, 85%, 86%, 87%, 88%, 89%, 90%, 91%, 92%, 93%, 94%, 95%, 96%, 97%, 98%, or 99% of homology. Random or engineered point mutation may be performed on the nucleotide and amino acid sequences in the present invention in an appropriate manner, with the aim of, for example, obtaining better expression level, affinity, and/or dissociation property. The mutated nucleotide or amino acid sequences fall within the scope of protection of the present invention.

Preferably, in some implementations of the present invention, the antigen-binding moiety may be Fab, Fab', F(ab')₂, Fd, Fv, scFv, or a single domain antibody (dAB), which can form an antigen-antibody complex structure with a target protein.

More preferably, in one implementation of the present invention, the antigen-binding moiety is scFv.

In some implementations of the present invention, the anti-CD7 scFv includes repeated numbers of VL and VH sequences, and the VL and VH sequences include the CDRs. The VL and VH may be connected by a suitable linker/linker peptide, which may be generally composed of glycine (Gly) and serine (Ser), such as GGGGS.

In some implementations of the present invention, the anti-CD7 scFv is a monovalent antibody. In other implementations of the present invention, the number of repeated sequences in the light chain variable region (VL) and the heavy chain variable region (VH) may be 2 or 3. When the number of repeat sequences is 2, the anti-CD7 scFv forms a diabody; or when the number of repeat sequences is 3, the anti-CD7 scFv forms a triabody. The light chain variable region (VL) and the heavy chain variable region (VH) may be connected in any suitable order, such as N-VL-VH-C or N-VH-VL-C.

Preferably, in some implementations of the present invention, the amino acid sequence of the light chain variable region of the monovalent scFv is as shown in SEQ ID No. 7, and the amino acid sequence of the heavy chain variable region of the monovalent scFv is as shown in SEQ ID No. 8, or a sequence having more than 80% of homology to the sequence.

More preferably, in one implementation of the present invention, the amino acid sequence of the monovalent scFv is as shown in SEQ ID No. 9, or a sequence having more than 80% of homology to the sequence.

The antigen-binding moiety specifically binding to CD7 may be obtained using a method well-known to those skilled in the art, such as chemical reagent treatment, or digestion with protease such as papain or pepsin, or construction of a vector for expression in an expression system, or synthesis.

In the present invention, the antibody may be prepared using the hybridoma preparation method reported by Kohler et al. in Nature 256:495 (1975). Mice or other suitable host animals are first immunized with an immunogen (added with an adjuvant if necessary).

The immunogen or adjuvant is generally injected by subcutaneous multi-point injection or intraperitoneal injection. The adjuvant may be Freund's adjuvant (Freund's complete adjuvant or Freund's incomplete adjuvant), MPL-TDM, or the like. After immunized, the animals produce lymphocytes in their bodies that secrete antibodies specifically binding to immunogens. Target lymphocytes are collected and fused with myeloma cells using an appropriate fusion agent (such as PEG4000) to obtain hybridoma cells (Goding, Monoclonal Antibodies: Principles and Practice, pp.59-103, Academic Press, 1996).

The prepared hybridoma cells are inoculated into a suitable medium for growth, where the medium contains one or more substances that can inhibit the growth of unfused maternal myeloma cells. For example, for maternal myeloma cells lacking hypoxanthine-guanine phosphoribosyl transferase (HGPRT or HPRT), the growth of HGPRT-deficient cells can be inhibited by adding substances such as hypoxanthine, aminopterin, and thymine (HAT medium) to the medium.

The preferred myeloma cells should have capabilities such as high fusion rate, stable antibody secretion ability, and sensitivity to the HAT medium. The myeloma cells are preferably mouse-derived myeloma, such as MOP-21 and MC-11 mouse tumor derived strains (THE Salk Institute Cell Distribution Center, San Diego, Calif. USA) and SP-2/0 or X63-Ag8-653 cell strains (American Type Culture Collection, Rockville, MD. USA).In addition, human myeloma and human-mouse allogeneic myeloma cell strains may also be used to prepare human monoclonal antibodies (Kozbor, J. Immunol., 133:3001 (1984); Brodeur et al., Monoclonal Antibody Production Techniques and Applications, pp.51-63, Marcel Dekker, Inc., New York, 1987).

The medium for the growth of hybridoma cells is used for testing the production of monoclonal antibodies targeting specific antigens. The binding specificity of monoclonal antibodies produced by hybridoma cells may be determined using the following methods: immunoprecipitation or in vitro binding tests, such as radio immunoassay (RIA) and enzyme-linked immunosorbent assay (ELISA). For example, the affinity of monoclonal antibodies may be determined using the Scatchard analysis method described by Munson et al. in Anal. Biochem. 107:220 (1980).

After the specificity, affinity, and reactivity of antibodies produced by hybridomas are determined, the target cell strains may be subcloned using the limited dilution method described in Goding, Monoclonal Antibodies: Principles and Practice, pp.59-103, Academic Press, 1996. The suitable medium may be DMEM, RPMI-1640, or the like.In addition, the hybridoma cells may grow in the form of ascitic tumors in animals.

The monoclonal antibodies secreted by the subcloned cells may be separated from cell culture fluid, ascites, or serum using a conventional immunoglobulin purification method, such as protein A agarose gel, hydroxyapatite chromatography, gel electrophoresis, dialysis, or affinity chromatography to obtain monoclonal antibodies.

Another aspect of the present invention is to provide a polynucleotide that encodes the aforementioned antibody or antigen-binding moiety.

Further, the polynucleotide sequence of the present invention may be inserted into any suitable expression vector in a suitable manner, such as bacterial plasmids, bacteriophages, yeast plasmids, plant cell viruses, mammalian cell viruses such as adenoviruses, retroviruses, or other vectors. Similarly, suitable host cells having genetic functions may include the polynucleotide or expression vector, such as T cells or NK cells expressing chimeric antigen receptors.

Another aspect of the present invention is to provide a vector including a sequence of the aforementioned polynucleotide.

Another aspect of the present invention is to provide an isolated cell including the aforementioned antibody, antigen-binding moiety, polynucleotide, or vector; and
the cell is selected from a SP2/0, YB2/0, IR983F, PERC6, or CHO cell line, or an insect cell line.

Another aspect of the present invention is to provide a use of the aforementioned antibody or antigen-binding moiety in preparing a product for testing or treating a tumor.

Preferably, in some implementations of the present invention, the tumor is a hematologic tumor.

More preferably, in some implementations of the present invention, the hematologic tumors are acute T lymphoblastic leukemia, T lymphoblastic leukemia/lymphoma, peripheral T cell lymphoma, vascular immunoblastic T cell lymphoma, anaplastic large cell lymphoma, or CD7 expressed positive acute myeloid leukemia.

The product for testing a tumor may be a reagent, a kit, or a cell culture plate for testing the CD7 content in a sample.

Another aspect of the present invention is to provide a pharmaceutical composition including the aforementioned antibody, or antigen-binding moiety, or isolated cell, or polynucleotide, and a pharmaceutically acceptable vector.

The pharmaceutical composition may be an oral preparation or an injection.

Another aspect of the present invention is to provide an immunoconjugate, including:
a) the aforementioned antibody or antigen-binding moiety;
b) a conjugating moiety selected from a drug, an enzyme, a detectable marker, a toxin, a cytokine, or a radionuclide; and
c) a linker for a) and b).

The antibody or antigen-binding moiety in the present invention may be conjugated with the conjugating moiety in any suitable manner to form a conjugate, such as an antibody-drug conjugate (ADC), for preventing or treating tumors.

Another aspect of the present invention is to provide a chimeric antigen receptor including an extracellular region, a transmembrane region, and an intracellular region, where the intracellular region includes an intracellular signal transduction region, the extracellular region of the chimeric antigen receptor includes a CD7-binding domain, and the CD7-binding domain includes the aforementioned antigen-binding moiety.

Preferably, in one implementation of the present invention, the amino acid sequence of the antigen-binding moiety is as shown in SEQ ID No. 9, or a sequence having more than 80% of homology to the sequence.

In some implementations of the present invention, the extracellular region further includes a signal peptide. The signal peptide may guide the transfer of antigen recognition and hinge regions to the extracellular space. Any suitable signal peptide or a combination of signal peptides can achieve the objective of the present invention.

Preferably, in some implementations of the present invention, the extracellular region further includes a signal peptide constructed at an amino terminal of the chimeric antigen receptor or an amino acid sequence having more than 90% of homology to the signal peptide; and

More preferably, in one implementation of the present invention, the signal peptide is of a signal peptide sequence in CD8α or is GM-CSF.

Further preferably, in one implementation of the present invention, the signal peptide is a signal peptide as shown in SEQ ID No. 10.

In some implementations of the present invention, the CD7-binding domain included in the chimeric antigen receptor of the present invention is connected to the transmembrane region encoded by the chimeric antigen receptor through a hinge region. Any suitable hinge region sequence can achieve the objective of the present invention. Preferably, in one implementation of the present invention, the hinge region is of a hinge region sequence in CD8α.

In some implementations of the present invention, the chimeric antigen receptor further includes a transmembrane domain. Any suitable transmembrane domain can achieve the objective of the present invention. Preferably, in some implementations of the present invention, the transmembrane region may be a transmembrane domain selected from the following proteins or an amino acid sequence having more than 90% of homology to the proteins: α, β, or ζ chain of a T cell receptor, CD2, CD3ε, CD4, CD7, CD8α, CD8β, CD11a, CD11b, CD11c, CD11d, CD18, CD19, CD27, CD28, CD29, CD30, CD40, CD48, CD49a, CD49d, CD49f, CD66a, CD66b, CD66c, CD66d, CD66e, CD69, CD79A, CD79B, CD84, CD96, CD100, CD103, CD134, CD137, CD150, CD158A, CD158B1, CD158B2, CD158C, CD158D, CD158F1, CD158F2, CD158K, CD160, CD162, CD226, CD229, CD244, CD247, CD258, CD268, CD270, CD272, CD276, CD279, CD314, CD319, CD335, CD336, CD337, CD352, CD353, CD355, CD357, LFA-1, NKG2C, DAP-10, ICAM-1, NKp80, IL-2R beta, IL-2Rgamma, IL-7R alpha, LFA-1, SLAMF9, LAT, GADS, SLP-76, PAG1/CBP, CD83 ligand, Fc gamma receptor, integrin, activating NK cell receptor, or Toll ligand receptor, or a combination thereof.

More preferably, in one implementation of the present invention, the transmembrane region is of a transmembrane region sequence in CD8α.

In some implementations of the present invention, the intracellular region included in the chimeric antigen receptor further includes a co-stimulatory factor;

Preferably, in some implementations of the present invention, the co-stimulatory factor may be one or more of functional signal domains obtained from the following proteins or amino acid sequences having more than 90% of homology to the proteins: integrin, BTLA, Toll ligand receptor, OX40, CD2, CD7, CD27, CD28, CD30, CD40, CDS, ICAM-1, LFA-1, 4-1BB, B7-H3, CD278, GITR, BAFFR, LIGHT, HVEM, KIRDS2, SLAMF7, NKp80, NKp44, NKp30, NKp46, CD19, CD4, CD8α, CD8β, IL2Rβ, IL2Rγ, IL7Rα, ITGA4, VLA1, CD49α, IA4, CD49D, ITGA6, VLA6, CD49f, ITGAD, CD11d, ITGAE, CD103, ITGAL, CD11α, ITGAM, CD11b, ITGAX, CD11c, CD29, ITGB1, ITGB2, CD18, ITGB7, NKG2D, NKG2C, TNFR2, CD226, CD84, CD96, CEACAM1, CRTAM, CD229, CD160, PSGL1, CD100, CD69, SLAMF6, SLAM, BLAME, CD162, LTBR, LAT, GADS, or SLP-76; and

More preferably, in one implementation of the present invention, the co-stimulatory factor is CD28 or 4-1BB, or an amino acid sequence having more than 90% of homology to the CD28 or 4-1BB.

In some implementations of the present invention, the chimeric antigen receptor further includes an intracellular signal transduction region. Preferably, in some implementations of the present invention, the intracellular signal transduction region may be selected from the following proteins or an amino acid sequence having more than 90% of homology to the proteins: 4-1BB, B7-H3, BAFFR, BLAME, BTLA, CD100, CD103, CD160, CD18, CD19, CD19a, CD2, CD247, CD27, CD276, CD28, CD29, CD3ζ, CD30, CD4, CD40, CD49a, CD49D, CD49f, CD69, CD7, CD84, CD8alpha, CD8beta, CD96, CDS, CEACAM1, CRTAM, DAP-10, DNAM1, Fc gamma receptor, GADS, GITR, HVEM, IA4, ICAM-1, ICAM-1, Ig alpha, IL2R beta, IL2R gamma, IL7R alpha, integrin, ITGA4, ITGA4, ITGA6, ITGAD, ITGAE, ITGAL, ITGAM, ITGAX, ITGB2, ITGB7, KIRDS2, LAT, LFA-1, LFA-1, LIGHT, LIGHT, LTBR, Ly9, NKG2C, NKG2D, NKp30, NKp44, NKp46, NKp80, OX-40, PAG/Cbp, PD-1, PSGL1, SELPLG, SLAMF4, SLAMF6, SLAMF7, SLP-76, TNFR2, Toll ligand receptor, TRANCE/RANKL, VLA1, or VLA-6, or a combination thereof.

More preferably, in one implementation of the present invention, the intracellular signal transduction region is CD3ζ.

Preferably, in some implementations of the present invention, the chimeric antigen receptor has a signal transduction domain having a structure formed by concatenation of a CD7 scFv antigen recognition region, a CD8α hinge region, a transmembrane region, a 4-1BB co-stimulatory molecule, and a Cd3ζ intracellular signal domain, and the tandem sequence of the signal transduction domain includes a nucleic acid sequence as shown in SEQ ID No. 12 and an amino acid sequence shown in SEQ ID No. 11.

In addition, any peptide chain may be inserted as a spacer at an appropriate position between the aforementioned antigen recognition region, hinge region, transmembrane region, and intracellular signal region, and the peptide chain may be of an oligopeptide or polypeptide.

Another aspect of the present invention is to provide a polynucleotide that encodes the aforementioned chimeric antigen receptor; and
Preferably, in one implementation of the present invention, the sequence of the polynucleotide is as shown in SEQ ID No. 12.

The nucleic acid molecule may be prepared by a known technology such as chemical synthesis or PCR amplification based on base sequences of the aforementioned antigen recognition region, hinge region, transmembrane region, intracellular signal region, etc. Generally, codons of amino acids that encode the aforementioned domains may be optimized to optimize their expression in host cells. Information about the base sequences may be obtained by searching known literature or databases such as NCBI (https://www.ncbi.nlm.nih.gov/).

In one implementation of the present invention, the inventor obtained the base sequence of the CD7 scFv antigen recognition region of the CD7-binding domain by PCR amplification. Specifically, total RNA of hybridoma cell strains containing mouse anti-human CD7 monoclonal antibodies was extracted, a first strand of cDNA was synthesized by reverse transcription, and mouse anti-human CD7 scFv was synthesized by PCR amplification with a "mouse antibody scFv gene amplification kit".

Another aspect of the present invention is to provide a vector including the aforementioned nucleic acid molecules.

In the present invention, the vector may be a straight chain vector or a cyclic vector. The vector may be a non-viral vector such as plasmids, a viral vector, or a vector using transposons. The vector may include regulatory sequences such as promoters and terminators, and marker sequences such as drug resistance genes and reporter genes. In addition, the vector may alternatively include sequences encoding suicide genes. The number of CAR-T cells in vivo may be controlled by administering substances that activate suicide genes in the treatment process.

The viral vector may be a retroviral vector, a lentiviral vector, an adenoviral vector, an adeno-associated viral vector, etc. In one implementation of the present invention, a lentiviral expression vector is used.

Another aspect of the present invention is to provide an isolated cell including the antigen-binding moiety, the chimeric antigen receptor, or a sequence of the polynucleotide as described above;
The cell is a T cell or an NK cell; and
Preferably, in some implementations of the present invention, the T cells are either autologous T cells or allogeneic T cells.

In some implementations of the present invention, the cells are human T cells. The T cells may come from body fluids such as blood and bone marrow, or tissues such as spleen, thymus, and lymph, or cancer tissues such as primary tumors, metastatic tumors, and cancerous ascites, and are obtained after isolation and purification. Preferably, the T cells are autologous T cells. Meanwhile, the T cells may be CD4⁺T cells, CD8⁺T cells αβT cells, or γδT cells, or a mixture of the aforementioned T cells. The T cells may be replaced with NK cells in an appropriate manner, which is also considered to fall within the scope of protection of the present invention.

In order to achieve an objective of the present invention, in some implementations of the present invention, the aforementioned cells are allogeneic T cells, and the T cells are universal chimeric antigen receptor T cells (CAR-T);
Preferably, in one implementation of the present invention, the T cells lack a CD7 gene sequence; and
More preferably, in one implementation of the present invention, the T cells also lack a *TRAC* gene sequence.

Another aspect of the present invention is to provide a method for constructing universal CAR-T cells, where the method involves introducing a nuclear protein complex targeting a *TRAC* gene into T cells by electroporation to achieve knockout of the *TRAC* gene and deficiency of TCRα/β antigen expression.

Another aspect of the present invention is to provide a method for constructing CD7^{-/-}CAR-T, where the method involves introducing a nuclear protein complex targeting a CD7 gene into T cells by electroporation to achieve knockout of the CD7 gene and deficiency of CD7 membrane antigen expression.

Preferably, in one implementation of the present invention, the aforementioned gene is knocked out using a CRISPR/Cas9 system, where the sequences of the gRNA (guideRNA) used are:
CD7 gRNA: GGGGTCAATGTCTACGGCTC
TRACgRNA: AGAGTCTCTCAGCTGGTACA

Another aspect of the present invention is to provide a use of the aforementioned antibody, antigen-binding moiety, polynucleotide, vector, isolated cell, or chimeric antigen receptor in preparing a drug for treating a tumor;
Preferably, in some implementations of the present invention, the tumor is a hematologic tumor;
More preferably, in some implementations of the present invention, the tumor is a T cell hematologic tumor;
Further preferably, in some implementations of the present invention, the tumor is CD7 antigen positive acute T lymphocytic leukemia or lymphoma; and
Further preferably, in one implementation of the present invention, the tumor is a refractory or relapsed CD7 antigen positive acute T lymphocytic leukemia or lymphoma.

Another aspect of the present invention is to provide a pharmaceutical composition including the aforementioned antibody, antigen-binding moiety, polynucleotide, chimeric antigen receptor, vector, or cell, and a pharmaceutically acceptable vector.

In addition to the aforementioned ingredients, the pharmaceutical composition of the present invention may further include any pharmaceutically permissible additive, such as physiological saline, cell medium, glucose, injection water, glycerol, ethanol, and a combination thereof, stabilizers, surfactants, preservatives, and isotonic agents.

Similarly, the pharmaceutical composition of the present invention may be used with other suitable anticancer agents, such as vincristine, daunorubicin, asparaginase, cyclophosphamide, and prednisone.

Preferably, in one implementation of the present invention, the aforementioned pharmaceutical composition further includes anti-CD5 CAR-T cells.

Another aspect of the present invention is to provide a method for treating a hematologic tumor by administering the aforementioned antibody, antigen-binding moiety, cell, or pharmaceutical composition to a patient.

Another aspect of the present invention is to provide a use of the aforementioned pharmaceutical composition in preparing a drug for treating a tumor;
Preferably, in some implementations of the present invention, the tumor is a hematologic tumor;
More preferably, in some implementations of the present invention, the tumor is a T cell hematologic tumor;
Further preferably, in some implementations of the present invention, the tumor is CD7 antigen positive acute T lymphocytic leukemia or lymphoma; and
Further preferably, in one implementation of the present invention, the tumor is a refractory or relapsed CD7 antigen positive acute T lymphocytic leukemia or lymphoma.

Beneficial effects of the present invention are as follows:
The anti-CD7 antibody and antigen-binding moiety have high affinity with CD7 protein and strong binding specificity. T cells that express a universal chimeric antigen receptor targeting CD7 are constructed. Experiments verify that the T cells modified by the chimeric antigen receptor have a strong killing effect on acute lymphoblastic leukemia (T-ALL) cell lines expressing CD7 and patient's primary cells expressing CD7, almost have no killing effect on cells that do not express CD7, and effectively prevent off-target effects. The chimeric antigen receptor CD7 scFv-CD8α-4-1BB-CD3ζ can be used for the treatment of CD7 antigen positive hematologic tumors and combined treatment with anti-CD5 CAR-T cells.

### BRIEF DESCRIPTION OF THE SEVERAL VIEWS OF THE DRAWINGS

FIG. 1 is a PCR amplification electrophoresis image of a light chain variable region (VL) and a heavy chain variable region (VH) of a mouse anti-human CD7 monoclonal antibody in an example of the present invention, where 1 is a 2Kbp nucleic acid molecular weight standard lane, 2 and 3 are VL lanes, and 4 and 5 are VH lanes;
FIG. 2 is an electrophoresis image of mouse-derived CD7 VL-VH (mLH) fragment sequences after gel extraction and purification and overlap PCR amplification on VL and VH sequences, where 1 is a 2Kbp molecular weight marker lane, and 2-5 are CD7 VL-VH fragment lanes;
FIG. 3 is a schematic structural diagram of CD7 targeted chimeric antigen receptor plasmids;
FIG. 4 is a statistical diagram of amplification folds of primary T cells that have not been knocked out or infected with a lentivirus (primary T), CAR-T cells that have not been knocked out but infected with a lentivirus (WT-7CAR), T cells that have been knocked out but not infected with a lentivirus (T-knockout), and CAR-T cells that have been knocked out and infected with a lentivirus (KO-7CAR);
FIG. 5 is a test result diagram of CD7CAR infection efficiency in T cells after TRAC and CD7 knockout;
FIG. 6 is a test diagram of CD7 and TCRα/β expression results of T cells in an experimental group, where A is a schematic result of CD7 and TCR α/βknockout efficiency in one experiment, and B is a statistical result of knockout efficiency in multiple experiments;
FIG. 7 is a statistical diagram of inhibitory receptor PD-1, LAG-3, and TIM-3 expression results of primary T, WT-7CAR, T-knockout, and KO-7CAR cells;
FIG. 8 is a statistical diagram showing the expression of activation related receptors CD25 and CD69 in primary T, WT-7CAR, T-knockout, and KO-7CAR cell;
FIG. 9 is a test result diagram of apoptosis induced by activation of primary T, WT-7CAR, T-knockout, and KO-7CAR cells (AICD);
FIG. 10 is a statistical result diagram of CD8 expression and classification of primary T, WT-7CAR, T-knockout, and KO-7CAR cells;
FIG. 11 is a test result diagram of CD7 antigen expression in a target cell line and patient's primary cells;
FIG. 12 is a summary diagram of test results of residual target cell proportions in 3 independent repeated experiments in which primary T, T-knockout, KO-7CAR cells were co-cultured with a target cell line in effector target ratios of 5:1 and 1:1 for 24 h and 48 h;
FIG. 13 shows co-culture test results of T/CAR-T cells and primary cells from 3 patients, where A represents residual target cell ratios after primary T, WT-7CAR, T-knockout, and KO-7CAR cells were co-cultured with primary cells from 3 patients in effector target ratios of 1:1, 1:2, and 1:4 for 24 h and 48 h, and B represents CD7 antigen expression test results of primary cells from patients after T/CAR-T cells were co-cultured with the primary cells from patients for 48 h;
FIG. 14 is a degranulation test result diagram of T/CAR-T cells, where A represents degranulation test results after primary T, T-knockout, and KO-7CAR cells were co-cultured with a target cell line, and B represents degranulation test results after primary T, WT-7CAR, T-knockout, and KO-7CAR cells were co-cultured with primary cells from patients;
FIG. 15 is an in vivo imaging test result diagram of small animals in a universal CAR-T in vivo experiment;
FIG. 16 is a binding result diagram of an antibody or antigen-binding moiety (APC fluorescence channel) of the present invention and a CD7-6B7 commercialized antibody (PE/Cy7 fluorescence channel) to CD7 antigen negative K562 and Raji cells;
FIG. 17 is a binding result diagram of an antibody or antigen-binding moiety (APC fluorescence channel) of the present invention and a CD7-6B7 commercialized antibody (PE/Cy7 fluorescence channel) to CD7 antigen positive Jurkat, Molt4, CEM, and Hut78 cell surface CD7 antigens; and
FIG. 18 is a result diagram of competitive binding of an antibody or antigen-binding moiety of the present invention and CD7-6B7 to a CD7 antigen tested by an FACS method.

### DESCRIPTION OF SEQUENCES

SEQID No. 1-3 are amino acid sequences of CDR1-3 in a light chain variable region of an antibody or antigen-binding moiety of the present invention;
SEQID No. 4-6 are amino acid sequences of CDR1-3 in a heavy chain variable region of the antibody or antigen-binding moiety of the present invention;
SEQID No. 7 is an amino acid sequence of a light chain variable region of a single chain antibody scFv in an embodiment of the present invention;
SEQID No. 8 is an amino acid sequence of a heavy chain variable region of the single chain antibody scFv in an embodiment of the present invention;
SEQID No. 9 is an amino acid sequence of the single chain antibody scFv in an embodiment of the present invention;
SEQID No. 10 is an amino acid sequence of a signal peptide in an embodiment of the present invention;
SEQID No. 11 is an amino acid sequence of a chimeric antigen receptor CD8α signal peptide - CD7 binding domain - CD8α hinge region - CD8α transmembrane region - 41BB co-stimulatory region - CD3ζ signal transduction region in an embodiment of the present invention;
SEQID No.12 is a nucleotide sequence of a chimeric antigen receptor CD8α signal peptide - CD7 binding domain - CD8α hinge region - CD8α transmembrane region - 41BB co-stimulatory region - CD3ζ signal transduction region in an embodiment of the present invention.

### DETAILED DESCRIPTION OF THE INVENTION

The present invention discloses an antibody specifically binding to CD7 and a use thereof in preparing a chimeric antigen receptor. Those skilled in the art can appropriately improve process parameters with reference to the content herein. It should be pointed out that all similar substitutions and modifications are obvious to those skilled in the art and are considered to be included in the present invention, and relevant personnel are clearly able to make modifications or appropriate changes and combinations to the content described herein without departing from the content, spirit, and scope of the present invention to implement and apply the technology of the present invention.

In the present invention, unless otherwise specified, the scientific and technical terms used herein have the meanings generally understood by those skilled in the art. The term "include" or its transformations such as "included" or "including" will be understood to include the stated elements or components throughout the specification and claims, and does not exclude other elements or components, unless otherwise expressly stated. The terms "such as" and "for example" are intended to indicate exemplary embodiments and are not intended to limit the scope of the present disclosure.

The following explains some terms that appear in the present invention.

The term "antibody" refers to an immunoglobulin molecule generally consisting of two pairs of polypeptide chains (each pair having a "light" (L) chain and a "heavy" (H) chain). Light chains of antibodies may be classified as κ and λ light chains. Heavy chains may be classified as µ, δ, γ, α, or ε chains, and isotypes of antibodies are defined as IgM, IgD, IgG, IgA, and IgE, respectively. Within the light and heavy chains, a variable region and a constant region are connected by a "J" region of approximately 12 or more amino acids, while the heavy chain further includes a "D" region of approximately 3 or more amino acids. Each heavy chain consists of a heavy chain variable region (V_{H}) and a heavy chain constant region (C_{H}). The heavy chain constant region consists of 3 domains (C_{H}1, C_{H}2, and C_{H}3). Each light chain consists of a light chain variable region (V_{L}) and a light chain constant region (C_{L}). The light chain constant region consists of a domain C_{L}. The constant region of the antibody may mediate binding of an immunoglobulin to a host tissue or factor, including various cells of an immune system (such as effector cells) and a first component of a classical complement system (C1q). The V_{H} and V_{L} regions may be further subdivided into regions with high variability (called complementary determining regions (CDRs)), interspersed with relatively conservative regions called framework regions (FRs). Each of VH and VL consists of 3 CDRs and 4 FRs arranged in the following order: FR1, CDR1, FR2, CDR2, FR3, CDR3, and FR4 from an amino terminal to a carboxyl terminal. The variable regions (V_{H} and V_{L}) of each heavy/light chain pair form an antibody binding site. The term "antibody" is not limited by any specific method for producing antibodies. For example, the antibody includes, in particular, recombinant antibodies, monoclonal antibodies, and polyclonal antibodies. The antibodies may be different types of antibodies, such as IgG (e.g., IgG1, IgG2, IgG3, or IgG4 subtypes), IgA1, IgA2, IgD, IgE, or IgM antibodies.

The term "antigen-binding moiety" refers to a polypeptide including a fragment of a full-length antibody, maintains the capability of specifically binding to the same antigen as the full-length antibody, and/or competing with the full-length antibody for specific binding to an antigen, and is also known as an "antigen-binding fragment". Usually refer to Fundamental Immunology, Ch.7 (Paul, W., ed., 2nd edition, Raven Press, N.Y. (1989), which is incorporated by reference herein for all purposes. The antigen-binding fragment of the antibody may be produced by recombinant DNA technology or enzymatic or chemical cleavage of the complete antibody. In some cases, the antigen-binding fragment includes Fab, Fab', F(ab')₂, Fd, Fv, etc.

The term "Fab fragment" refers to an antibody fragment consisting of V_{L}, V_{H}, C_{L}, and C_{H}1 domains; and the term "F(ab')₂ fragment" refers to an antibody fragment including two Fab fragments connected by a disulfide bridge on the hinge region. The term "Fd fragment" refers to an antibody fragment consisting of V_{H} and C_{H}1 domains; and the term "Fv fragment" refers to an antibody fragment consisting of V_{L} and V_{H} domains of a single arm of the antibody.

Herein, unless otherwise specified, when referring to the term "antibody", it includes not only the complete antibody but also the antigen-binding fragment of the antibody.

The term "specific binding" refers to a non-random binding reaction between two molecules, such as a reaction between an antibody and an antigen that the antibody targets.

The term "variable region" of the antibody refers to a variable region of the light chain or heavy chain of the antibody, either alone or in combination. As known in the art, the variable regions of the heavy and light chains each consist of 4 framework regions (FR) connected by 3 complementary determining regions (CDRs) also known as high variable regions. The CDRs in each chain are tightly held together through FR and with those from other chains to form antigen binding sites of the antibody. There are at least two techniques used for determining CDRs: (1) a method based on cross species sequence variation (i.e., Kabat et al. Sequences of Proteins of Immunological Interest, (5th ed., 1991, National Institutes of Health, Bethesda MD)); and (2) a method based on antigen-antibody complexes for crystallographic studies (Al-lazikani et al., 1997, J.Molec. Biol. 273:927-948). As used herein, the CDRs may refer to CDRs defined by either method or a combination of the two methods.

The term "co-stimulatory" refers to a secondary signaling event that activates an immune response for immune cells. The immune cells rely on co-stimulation to activate the immune response in the presence of antigen-presenting cells. For T cells, two stimuli are required to fully activate their immune response. That is, in the process of lymphocyte activation, co-stimulation is usually crucial for the development of an effective immune response. In addition to an antigen-specific signal from their antigen receptor, co-stimulation is also required. The second signal prevents activated T cells from being unresponsive, so that the T cells produce additional lymphokines necessary for the growth of the T cells.

In order to enable those skilled in the art to better understand the technical solution of the present invention, the present invention will be further described in detail below in conjunction with specific examples.

### Example 1: Preparation of CD7-HIT7 monoclonal antibody

### 1. Screening of mouse hybridoma monoclonal antibody

Balb/c mice were immunized by intraperitoneal injection with human CD7 protein as an immunogen, and booster immunization was performed in the 3^{rd} and 5^{th} weeks after initial immunization. Tail blood was collected from the mice on the 8^{th} day after the booster immunization, placed at room temperature for 1 hour, and centrifuged at 4°C and 12000 rpm for 10 minutes. Serum was collected and diluted with PBS to different concentrations: 1:200, 1:400, 1:800, 1:1600, 1:3200, 1:6400, and 1:12800. Jurkat cells were collected, washed with PBS once, and counted, then 1×10⁶ cells per sample were used. 100 µl of serum with different dilutions was added to the cells. The antiserum was replaced with serum from non-immunized mice in a negative control group. The cells were incubated at 4°C for 1 hour and washed with PBS twice. 2 µl of PE labeled rat anti-mouse IgG antibody was added, and the cells were incubated at 4°C in dark for 40 minutes. The cells were resuspended in 500 µl of PBS buffer, and the binding percentage and fluorescence intensity of the antibody to cells in the serum were tested by a flow cytometer. Cell fusion can only be performed when the effective titer was at least twice the average fluorescence intensity as negative control and the titer was more than 6400. 3 days before fusion, impact immunization was performed on the immunized mice by tail vein injection with an immunogen. Successfully immunized mouse spleen cells were fused with myeloma SP2/0 cells (in a 10: 1 ratio). During fusion, 50% PEG was added to the mixture of spleen and myeloma cells within 1 minute and mixed uniformly in a 37°C water-bath followed by shaking in the 37°C water-bath for 1 minute. Then 10 ml of serum-free 1640 medium was added within 2 minutes. Centrifugation was performed at 800 rpm for 6 minutes, the supernatant was discarded, the cells were resuspended in a 1640 medium containing HAT, and the solution was pipetted into a 96-well plate (2.5×10⁷ cells/plate). The cells were cultured under conditions of 37°C, 5*%* CO₂. When clones in the fusion plate were large enough, 100 µl of supernatant was taken from each well and co-incubated with 2×10⁵ Jurkat cells for testing using the same method as titer testing. The well having an average immunofluorescence intensity more than twice that of the negative well was used as a positive well for next clone culture. The screened positive hybridoma clones were cultured for 3-5 days in a 24-well plate instead of the 96-well plate, culture supernatant screening and test were performed again, positive clones were tested for next subclone culture, and the remaining cells were frozen. Hybridoma cells were collected from the 24-well plate and counted, and the cell density was adjusted to 10 cells/mL. The cells were spread into a 96-well plate, with 100 µl per well, and the cells were incubated in a 37°C, 5*%* CO₂ incubator. After about 10 days of culture, formed clones can be seen. Wells with only single clones were selected, the culture supernatant was extracted and tested by the same method as before, and positive clones were cultured in a 24-well plate. After further supernatant test, positive clones were selected for the second round of subclone culture. Generally, after multiple rounds of subclone culture, all tested wells were positive, and stable hybridoma cell strains were obtained. The positive hybridoma culture supernatant was selected, and the subtype of the antibody was tested with an antibody subtype test paper. The monoclonal antibody in the present invention was numbered HIT7, which was a mouse-derived IgG1 subtype and has a κ chain as a light chain.

### 2. Preparation and purification of ascites

The hybridoma cells were washed with a sterile PBS solution and injected intraperitoneally into Balb/c mice pre-sensitized with liquid paraffin in a cell volume of 5×10⁶/0.5ml/mouse. Ascites was collected 7 to 10 days later, and the supernatant was collected at room temperature and 3000 rpm for 10 minutes. The antibody was crudely purified with saturated ammonium sulfate having a final concentration of 33% by the following method: 1 part of ascites was added to 1 part of PBS, 1 part of saturated ammonium sulfate was added dropwise while stirring, the solution was placed overnight at 4°C and centrifuged at 10000 rpm for 10 minutes to remove the supernatant, and the precipitate was dissolved in a small amount of PBS, followed by dialysis and desalting with PBS at 4°C for 24 hours during which the medium was changed 3 times. The crudely purified antibody was further purified using an AKTA protein purification system and a 1ml Protein G purified pre-packed column. The obtained pure antibody was used for subsequent antibody test and functional experiments.

3. By protein sequencing, the CDR1, CDR2, and CDR3 sequences of the light chain variable region of the monoclonal antibody HIT7 were as shown in SEQ ID No. 1-3, and the CDR1, CDR2, and CDR3 sequences of the heavy chain variable region were as shown in SEQ ID No.4-6.

### Example 2: Cloning of antigen recognition region CD7scFv in chimeric antigen receptor

1) Total RNA of the mouse anti-human CD7 monoclonal antibody hybridoma cell strains prepared in Example 1 was extracted: 1 ml of RNA iso Plus (Takara) was added to 5×10⁶ cells, followed by suspending and mixing well. 200 µl of trichloromethane was added, followed by up-and-down reversal and well vortex shaking. Centrifugation was performed at 4°C and 12000 rpm for 5 minutes. The supernatant was pipetted into a 1.5ml EP tube, and the same volume of isopropanol was added, followed by gentle up-and-down reversal and well mixing. Centrifugation was performed at 4°C and 12000 rpm for 15 minutes. 75% ethanol precipitate RNA was pre-cooled at 4°C, and total RNA was dissolved in 50 µl of DEPC water.
2) A first strand of cDNA was synthesized by reverse transcription: A PCR reaction system (20 µl) was prepared as follows: Oligo d (T) 15 Primers: 2 µl; M-MLV (200 u/µl): 1 µl; dNTP (each 2.5 mM): 1 µl; DTT (0.1 M): 2 µl; First strand buffer (5×): 4 µl; BCMA-RNA: 2 µg; DEPC water: made up to 20 µl. Reaction conditions: 37°C and 60 minutes, 70°C and 10 minutes.
3) Gene fragments of the light chain (VL) and the heavy chain (VH) of the mouse anti-human CD7 monoclonal antibody were amplified by PCR with a "mouse antibody scFv gene amplification kit" (Public Protein/Plasmid Library):

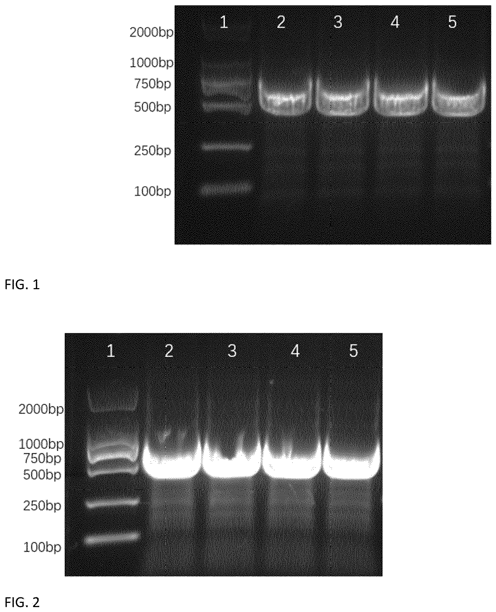

The VL chain was amplified: A PCR reaction system (50 µl) was prepared as follows: MVL mix: 45 µl; DNA polymerase: 0.3 µl; cDNA: 400 ng; ddH₂O: made up to 50 µl. Reaction conditions: pre-denaturation at 94°C for 3 minutes; the following cycle was repeated 30 times: 94°C for 30 seconds, 56°C for 30 seconds, 72°C for 45 seconds; extension at 72°C for 10 minutes;
The VH chain was amplified: A PCR reaction system (50 µl) was prepared as follows: MVH mix: 45 µl; DNA polymerase: 0.3 µl; cDNA: 400 ng; ddH₂O: made up to 50 µl. Reaction conditions: pre-denaturation at 94°C for 3 minutes; the following cycle was repeated 30 times: 94°C for 30 seconds, 56°C for 30 seconds, 72°C for 45 seconds; extension at 72°C for 10 minutes;
The VL and VH fragments were separated by agarose gel electrophoresis and extracted. The results were shown in FIG. 1.

4) The VL and VH were connected to a pMD19-simple T vector and sequenced, and the nucleic acid sequences of the VL and VH of the BCMA monoclonal antibody were determined according to the sequencing results. Humanization simulation was performed using computer software to obtain humanized VL and VH nucleic acid sequences, which were then synthesized.

5) CD7 scFv fragments in a VL-linker-VH direction were constructed by an overlap PCR method:
P1: CTAGCTAGCGACATTGTGCTGACACAGTCTCC
P2:GGTGGTGGTGGTTCTGGCGGCGGCGGCTCCGGTGGTGGTGGTTCTCAG GTGCAGCTGCAGGAGTC
P3:AGAACCACCACCACCGGAGCCGCCGCCGCCAGAACCACCACCACCAGC CCGTTTTATTTCCAGCTTGG
P4:CCGGAATTCCTGAAGAGACGGTGACCGTG

A first round of PCR reaction system was prepared to obtain an Nhe I -VL-linker fragment (50 µl):
2 × Pfu PCR Master Mix (TianGen Company): 25 µl;
10 µMP1 + P2: 2 µl;
PMD19-VL plasmids: 100 ng;
ddH₂O: made up to 50 µl.

Reaction conditions: pre-denaturation at 94°C for 5 minutes; the following cycle was repeated 32 times: 94°C for 30 seconds, 60°C for 30 seconds, 72°C for 45 seconds; finally, extension at 72°C for 10 minutes;
A first round of PCR reaction system was prepared to obtain a linker-VH-EcoR I fragment (50 µl):
2 × Pfu PCR Master Mix (TianGen Company): 25 µl;
10 µMP3 + P4: 2 µl;
pMD19-VH plasmids: 100 ng;
ddH₂O: made up to 50 µl.

Reaction conditions: pre-denaturation at 94°C for 5 minutes; the following cycle was repeated 32 times: 94°C for 30 seconds, 60°C for 30 seconds, 72°C for 45 seconds; extension at 72°C for 10 minutes;
The Nhe I -VL-linker and linker-VH-EcoR I fragments were separated by agarose gel electrophoresis and recovered;
A second round of PCR reaction system was prepared to obtain a CD7 scFv (VL-linker-VH direction) fragment (50 µl):
   2 × Pfu PCR Master Mix (TianGen Company): 25 µl;
   10 µMP1 + P4: 2 µl;
   Nhe I -VL-linker fragment: 100 ng;
   linker-VH-EcoR I fragment: 100 ng;
   ddH₂O: made up to 50 µl.

Reaction conditions:
pre-denaturation at 94°C for 5 minutes; the following cycle was repeated 32 times: 94°C for 30 seconds, 58°C for 90 seconds; extension at 72°C for 10 minutes.

The CD7 scFv fragments in the VL-linker-VH direction were separated by agarose gel electrophoresis and recovered, and the results were shown in FIG. 2.

The sequences described in this example may alternatively be commercially synthesized.

### Example 3: Construction of CD7 targeted chimeric antigen receptor

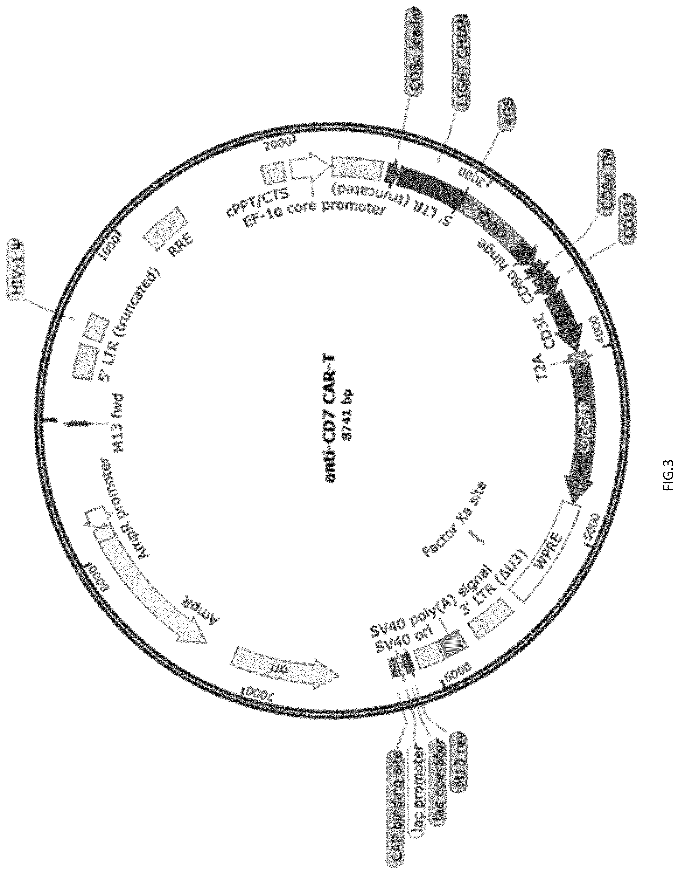

1) Vector plasmids containing CD8α-4-1BB-CD3ζ fragments (the plasmids were pre-constructed in the laboratory) were digested with Nhe I and EcoR I endonucleases to obtain vector fragments containing CD8α-4-1BB-CD3ζ. The plasmids containing CD8α-4-1BB-CD3ζ fragments can be prepared by any suitable method in existing technologies.
2) The CD7 scFv (mLH) and CD7 scFv (mHL) fragments obtained in Example 1 were connected to a target vector, the constructed CD7-VL-VH- CD8α-4-1BB-CD3ζ CAR target vector was identified by digestion with Nhe I and Not I, and the bacterial solution was sent for sequencing confirmation. If the sequence alignment was correct, the construction of plasmids was successful. The plasmid structure diagram was as shown in FIG. 3.

### Example 4: Preparation and transfection of lentivirus

1) Extraction of plasmids:
The above sequencing correct bacterial solution was added to a fresh LB liquid medium (20-30 ml) containing ampicillin in a ratio of 1:100 and cultured for 12-16 hours while shaking at a rate of 200 rpm/min in a 37°C constant temperature shaker, and plasmids were extracted with a plasmid extraction kit of TIANGEN Company;
1. Column equilibration step: 500 µl of equilibrium solution BL was added to an adsorption column CP4 (the adsorption column was put into a collection tube), centrifugation was performed at 12,000 rpm (~13,400 g) for 1 minute, the waste liquid in the collection tube was discarded, and the adsorption column was placed back into the collection tube.
2. The bacterial solution was added to a centrifuge tube and centrifuged at 12,000 rpm (~13,400 g) for 1 minute, and the supernatant was discarded as much as possible.
3. 500 µl of solution P1 (added with RNaseA) was added to the centrifuge tube containing bacterial cell precipitate, and the bacterial cell precipitate was thoroughly suspended using a pipette or a vortex oscillator.
4. 500 µl of solution P2 was added to the centrifuge tube, which was then gently turned up and down 6-8 times to fully lyse bacterial cells.
5. 500 µl of solution P4 was added to the centrifuge tube, which was then immediately gently turned up and down 6-8 times for well mixing, showing white flocculent precipitate. Afterwards, the bacterial solution was placed at room temperature for about 10 minutes and centrifuged at 12,000 rpm (~13,400 g) for 10 minutes.
6. The supernatant collected in the previous step was added in stages to a filter column CS (the filter column was put into the collection tube) and centrifuged at 12,000 rpm (~13,400 g) for 2 minutes, and the filtrate was collected in a clean 2 ml centrifuge tube.
7. Isopropanol 0.3 times the volume of the filtrate was added to the filtrate, which was then mixed upside down and pipetted to an adsorption column CP4 (the adsorption column was placed in the collection tube). The adsorption column CP4 had a maximum volume of 700 µl, and the filtrate was introduced to the column in stages as needed.
8. Centrifugation was performed at room temperature and 12,000 rpm (~13,400 g) for 1 minute, the waste liquid in the collection tube was discarded, and the adsorption column was placed back into the collection tube.
9. 500 µl of deproteinized solution PD was added to the adsorption column CP4, centrifugation was performed at 12,000 rpm (~13,400 g) for 1 minute, the waste liquid in the collection tube was discarded, and the adsorption column CP4 was put into the collection tube.
10. 600 µl of rinsing solution PW (added with anhydrous ethanol) was added to the adsorption column CP4, followed by standing at room temperature for 2-5 minutes and centrifuging at 12,000 rpm (~13,400 g) for 1 minute. The waste liquid in the collection tube was discarded, and the adsorption column CP4 was put into the collection tube.
11. 600 µl of rinsing solution PW was added to the adsorption column CP4, centrifugation was performed at 12,000 rpm (~13,400 g) for 1 minute, and the waste liquid in the collection tube was discarded.
12. The adsorption column CP4 was placed back into the collection tube, centrifugation was performed at 12,000 rpm (~13,400 g) for 2 minutes, and the adsorption column CP4 was opened and placed at room temperature for a few minutes to thoroughly dry the residual rinsing solution in the adsorption material.
13. The adsorption column CP4 was placed in a clean centrifuge tube, 100-300 µl of elution buffer TB or sterile double distilled water was added dropwise to the middle part of an adsorption membrane, the adsorption column was placed at room temperature for 2 minutes, centrifugation was performed at 12,000 rpm (~13,400 g) for 1 minute, and the plasmid solution was collected into the centrifuge tube.
14. The extracted plasmid solution was determined for concentration, A260/A280, and A260/A230 using NanoDrop2000.
15. The plasmid solution was stored at -20°C for subsequent transfection use.

2) Preparation and concentration of lentivirus:
1. 293T cells in a logarithmic growth phase were digested with pancreatin, and 5×10⁶ HEK293T cells were seeded into a 10 cm cell culture dish (DMEM + 15*%* FBS medium), with a total volume of 10 ml. The cells were shaken evenly and then the cell culture dish was placed in a 37°C, 5*%* CO₂ incubator to culture the cells for 24-36 hours. When the confluence of the cells reached 80-90%, transfection was performed.
2. DNA was diluted with a serum-free DMEM medium in a 15 ml centrifuge tube. The transfection system was as follows:

| Name | Dosage |
|---|---|
| Plasmids | 16 µg |
| pMD2.G | 3.5 µg |
| pMDLg/pRRE | 5.33 µg |
| PRSV-Rev | 2.51 µg |
| PEI | ~70 µl |
| Serum-free DMEM medium | 2.4 ml |

The above mixed system was placed for 15 minutes after being prepared. The old medium was pipetted from the 10 cm cell culture dish, 7 ml of fresh DMEM medium containing 15% FBS was added, the above transfection system was slowly added dropwise to the culture dish, the culture dish was placed in the 37°C, 5*%* CO₂ incubator for culture, and the medium was changed with completely fresh DMEM medium containing 15% FBS after 12 hours.
3. The stock virus solution was collected 48 hours after the medium was changed, the stock virus solution was centrifuged at 4°C and 3000 rpm for 15 minutes, and the supernatant was collected.
4. The virus supernatant was filtered with a 0.45 µm filter, collected into an ultra-centrifugation tube, balanced strictly with a precision balance, and centrifuged with an ultra-centrifuge at 4°C, 50000 g, 2 h 30 min.
5. After centrifugation, the supernatant was discarded, 100 µl of serum-free medium (KBM581) was added to resuspend the virus, and the virus was dispensed in 1.5 ml EP tubes and stored at -80°C for later use.

### Example 5: Knockout of CD7 and TRAC genes in T cells using CRISPR technology

1) Preparation of T cells:
   10 ml of fresh healthy human peripheral blood was collected, and T cells were isolated using RosetteSep T cell enrichment Cocktail (Stemcell Company) and Ficoll-Paque PLUS (GE Healthcare Company) (specific steps followed the instructions of RosetteSep T cell enriched Cocktail). Anti-CD3/CD28 magnetic beads (Gibco Company) were added in a 1: 1 ratio of cells: magnetic beads, and the cells were cultured for 48 hours to obtain T cells before gene knockout.
2) Knockout of CD7 and TRAC genes in T cells:
   1. The The T cells cultured after 48 hours were stimulated with anti-CD3/CD28 magnetic beads, the magnetic beads were removed after centrifugation, 3 ×10⁶ T cells were counted, excess DPBS was added, and centrifugation was performed at 1500 rpm for 10 minutes to wash the cells;
   2. Two 200 µl centrifuge tubes were taken and added with 3 µg CD7 gRNA + 3 µg Cas9 protein and 3 µg TRACgRNA + 3 µg Cas9 protein separately (gRNA sequences were previously screened in the laboratory with high knockout efficiency, prepared through a TAKARAGuide-it^{™} sgRNA In Vitro Transcription Kit, the Cas9 proteins were TakaraGuide-it^{™} Recombinant Cas9). Incubation with a PCR amplifier: 37°C, 5 minutes, followed by maintenance at 4°C.
      CD7 gRNA: GGGGTCAATGTCTACGGCTC
      TRACgRNA: AGAGTCTCTCAGCTGGTACA
   3. A Lonza 2b electroporator was used, and the electroporation kit was Human T Cell Nucleofector^{™} Kit. 100 µl of electroporation solution was prepared according to the operation instructions for immediate use.
   4. The cells were resuspended with 100 µl of electroporation solution and mixed with the incubated gRNA and Cas9 protein complex, and the mixture was added to an electroporation cup and transferred to the electroporator for electroporation.
   5. The electroporated cell solution was carefully pipetted to a T cell medium pre-heated at 37°C and placed in the 37°C, 5*%* CO₂ incubator for continued culture.

### Example 6: Lentiviral infection and amplification of T cells

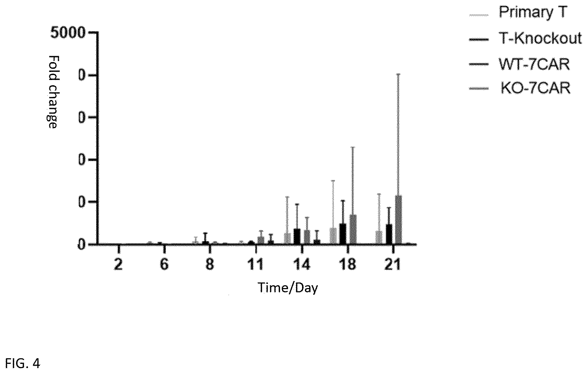

1) Infection of T cells with a lentivirus and culture of infected T cells: 4-6 hours after electroporation, the virus supernatant was taken out from -80°C and melted on ice, 100 µl of virus supernatant was added to per (0.5-1) × 10⁶ T cells, and Polybrene was added to a final concentration of 8 µg/ml. Centrifugation: 32°C, 1800 rpm, 1.5 h. After centrifugation, the culture well plate was placed in a 5*%* CO₂, 37°C incubator for culture.
2) Primary T cells that had not been knocked out or infected with a lentivirus (primary T), CAR-T cells that had not been knocked out but infected with a lentivirus (WT-7CAR), T cells that had been knocked out but not infected with a lentivirus (T-knockout), and CAR-T cells that had been knocked out and infected with a lentivirus (KO-7CAR) were collected, and T cells were counted on days 2, 6, 8, 11, 14, 18, and 21 to calculate cell amplification. FIG. 4 is a statistical diagram of amplification fold of the above cells. As shown in the figure, the anti-CD7 CAR-T cells without their CD7 being knocked out can not proliferate, while the anti-CD7 CAR-T cells with their CD7 being knocked out can continue to amplify until the 21st day, with thousands of times amplification on the 21st day. There was no difference in the amplification of T cells with and without TRAC and CD7 knockout, indicating that gene knockout had no significant effect on cell amplification. The CAR-T preparation strategy was feasible.

### Example 7: Test on phenotypes of universal CAR-T cells

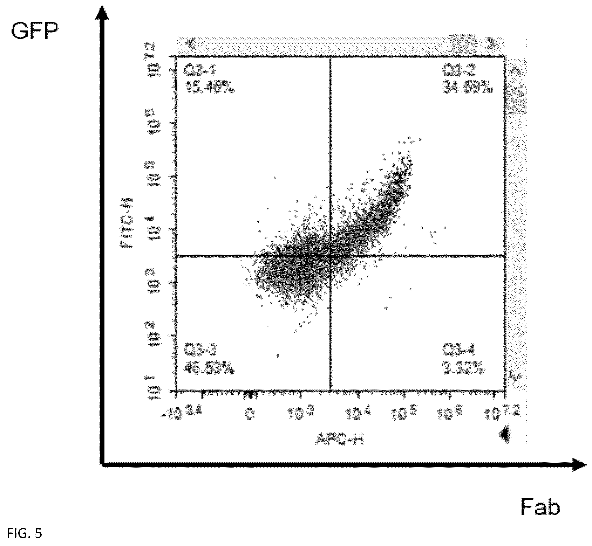

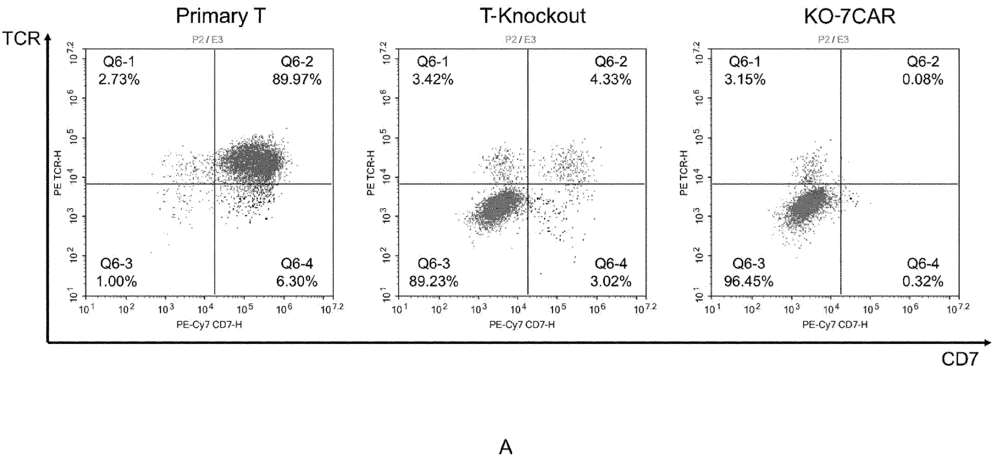

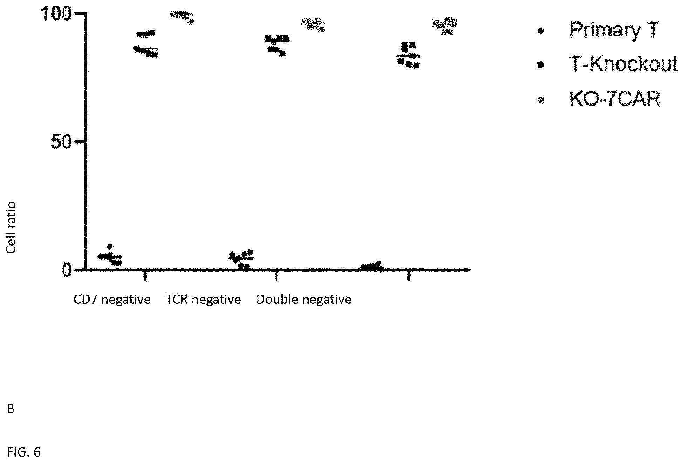

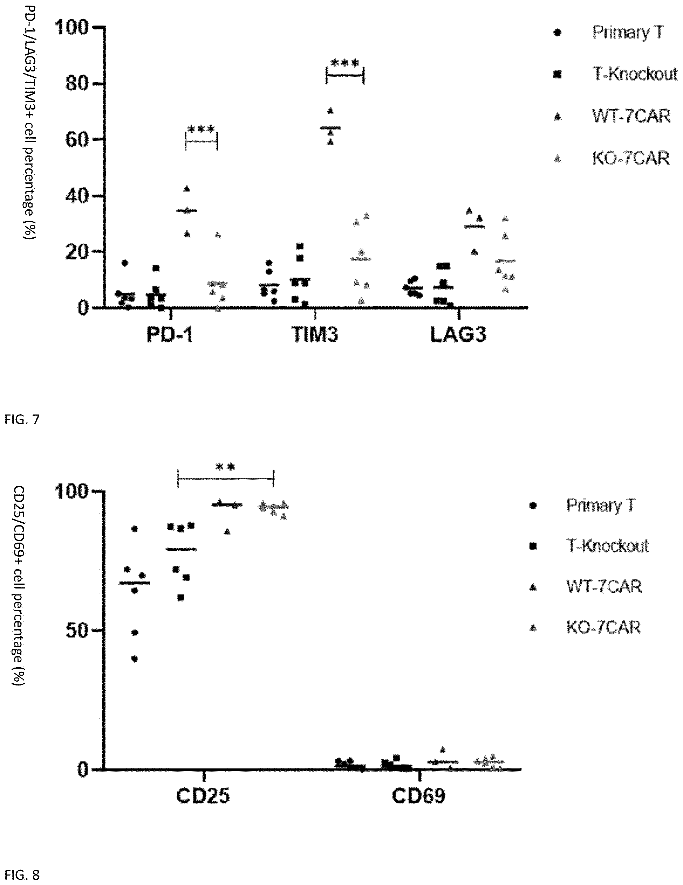

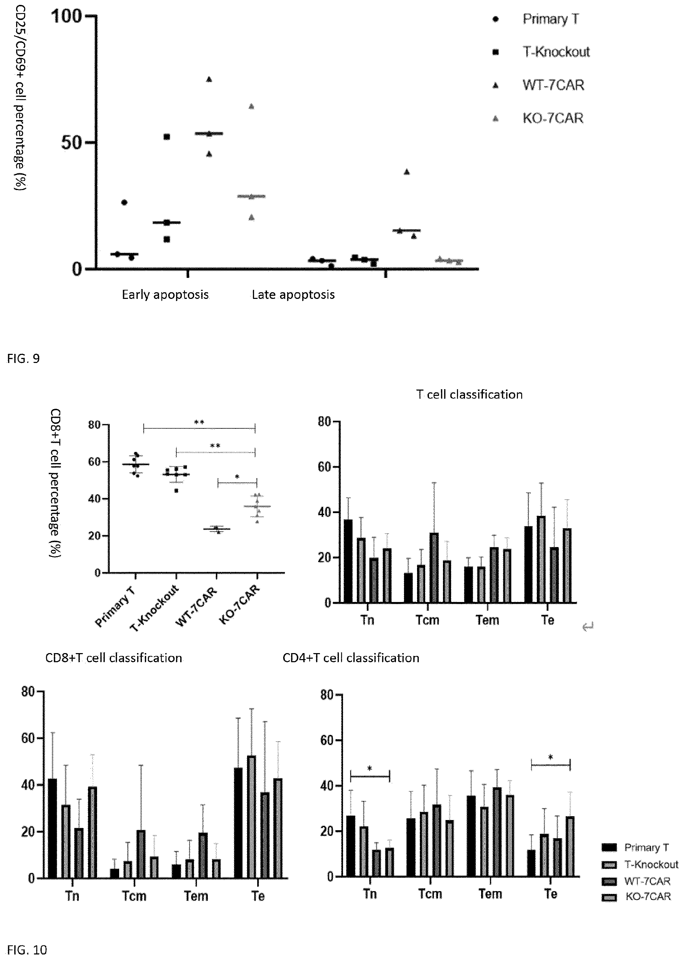

On the 7^{th} day of T cell culture (5^{th} day after gene knockout and lentiviral infection), ~1 × 10⁵ cells/tube were indicating for phenotype test by flow cytometry as follows:
1) Test on CAR expression and CD7 and TCR α/βknockout efficiency of T/CAR-T cells:
   Cells were collected, labeled with goat anti-mouse IgG F(ab')₂ antibody (APC test channel), incubated at room temperature for 15 minutes, washed twice with excess PBS, then labeled with anti-human PE/Cy7 CD7 and anti-human PE TCR α/β flow cytometry antibodies, incubated at room temperature for 15 minutes, washed with excess PBS, and resuspended for flow cytometry analysis. FIG. 5 shows the infection efficiency results of T cells, where KO-7CAR infection efficiency fluctuates between 15% and 40%. FIG. 6 shows CD7 and TCR α/β expression results, A in FIG.6 is a representative flow cytometry diagram of knockout efficiency in one experiment, and B in FIG. 6 is a statistical result of knockout efficiency in multiple experiments, showing that the proportions of CD7⁻TCRα/β⁻ cells after knockout were all above 90%.
2) Test on T/CAR-T cell inhibitory receptor expression:
   Cells were collected, labeled with anti-human APC/Cy7 PD-1, anti-human PE TIM3, and anti-human PE/Cy7 LAG3 flow cytometry antibodies, incubated at room temperature for 15 minutes, washed with excess PBS, and resuspended for flow cytometry analysis. FIG. 7 is a statistical diagram of inhibitory receptor expression results of primary T, WT-7CAR, T-knockout, and KO-7CAR. As shown in the figure, the inhibitory receptor expression of WT-7CAR without CD7 knockout was significantly increased, mainly mainly manifested by an increase in the expression of PD-1 and TIM-3, and a slight increase in the expression of LAG-3, but no significant statistical difference was reached.
3) Test on T/CAR-T cell activation receptor expression:
   Cells were collected, labeled with anti-human CD25 (late activation marker antibody for T cells) and anti-human CD69 (early activation marker antibody for T cells) flow cytometry antibodies, incubated at room temperature for 15 minutes, washed with excess PBS, and resuspended for flow cytometry analysis. FIG. 8 is a statistical diagram of expression results of primary T, WT-7CAR, T-knockout, and KO-7CAR activation related receptors. As shown in the figure, the CD25 expression of the anti-CD7 CAR-T cells was significantly higher than that of T cells (primary T, T-knockout), and there was no significant difference in the expression of activation related receptors between WT-7CAR and KO-7CAR.
4) Test on T/CAR-T cell activation induced apoptosis (AICD):
   Cells were collected and washed with 1 ml of AnnexinV binding buffer, then the supernatant was discarded, and the cells were resuspended with 100 µl of AnnexinV binding buffer, labeled with an AnnexinV antibody, incubated at room temperature for 15 minutes, added 1 µl of 500 µg/ml PI, mixed well, and then loaded at a low speed for flow cytometry. FIG. 9 shows AICD test results. As shown in the figure, the early apoptosis and late apoptosis of KO-7CAR were lower than those of WT-7CAR.
5) Test on CD8 expression and CD45RA and CCR7 cell classification of T/CAR-T cells:
   Cells were collected, labeled with anti-human PerCPCD8, anti-human APC/Cy7 CD45RA, and anti-human PECCR7 flow cytometry antibodies, incubated at room temperature for 15 minutes, washed with excess PBS, and resuspended for flow cytometry analysis. FIG. 10 shows statistical results of CD8 expression and cell classification of T/CAR-T cells. As shown in the figure, the CD8 expressions of WT-7CAR and KO-7CAR were lower than that of primary T cells. Overall, there was no significant difference in cell subtype among the groups.

### Example 8: Functional test on universal CAR-T cells

### 1) Test on CD7 antigen expression in target cells:

Jurkat, Molt4, Hut78, and CEM cell lines were selected as killer target cells (the target cells over-expressed a truncated CD19 antigen for testing), and the CD7 expression negative K562 cell line (the K562 cell line over-expressed a red fluorescent protein RFP for testing) was selected as control group cells. The cells were labeled with a CD7 antibody and an isotype control, incubated at room temperature for 15 minutes, washed with excess PBS, and resuspended for flow cytometry.

Primary cells from T-ALL patients were thawed, labeled with the CD7 antibody and the isotype control, incubated at room temperature for 15 minutes, washed with excess PBS, and resuspended for flow cytometry.

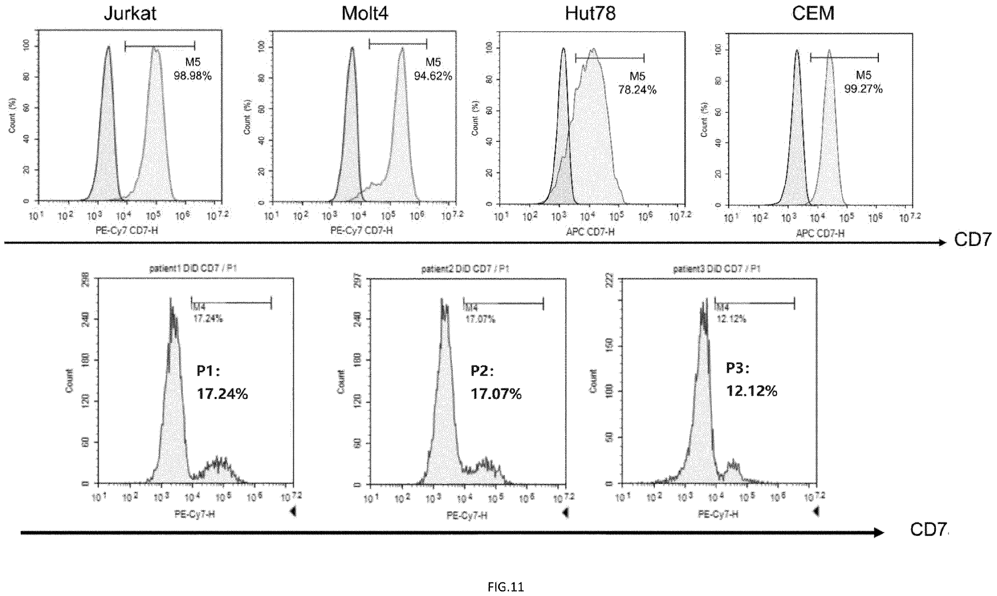

The CD7 antigen expression results of the target cell lines and the patients' primary cells are shown in FIG. 11.

### 2) CAR-T cells' killing function experiment:

### 1. Co-culture of T/CAR-T cells with target cell lines

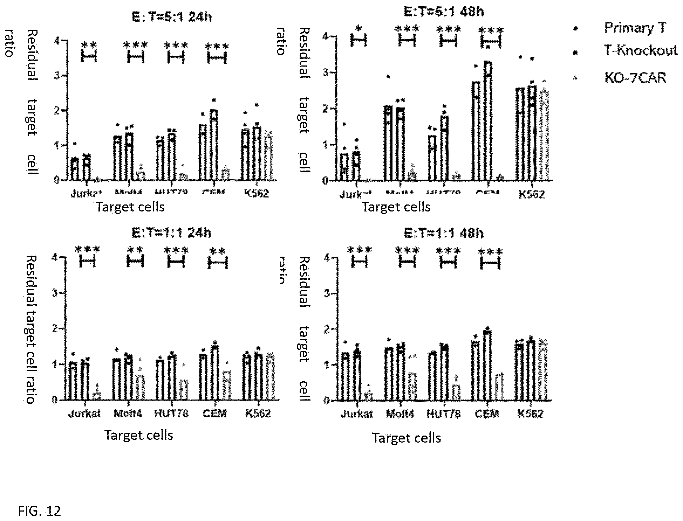

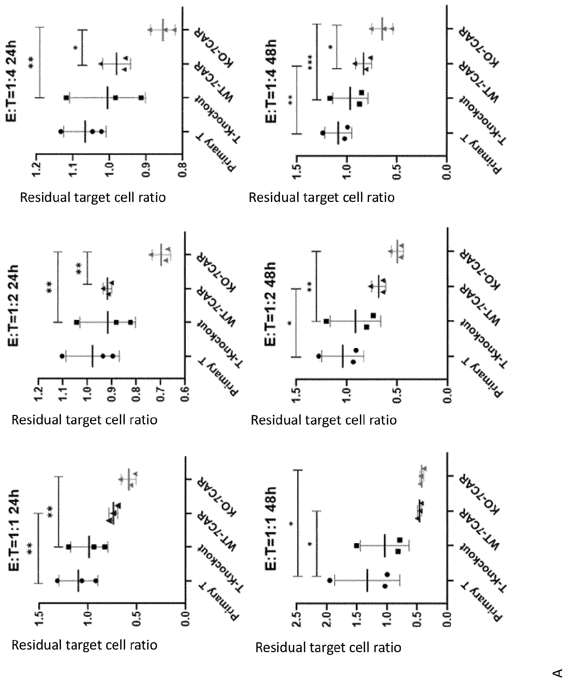

The Jurkat, Molt4, CEM, Hut78, and K562 cell lines were seeded into 48-well culture plates (total culture system 500 µl) at 5×10⁴ cells/well, and 2.5×10⁵ (E: T = 5: 1) and 5×10⁴ (E: T = 1: 1) of CAR modified T cells were added respectively. Primary T and T-knockout were used as control groups. The cells were co-cultured in an incubator. The co-cultured cells were labeled with an anti-human PE CD19 monoclonal antibody (Biolegend Company) by flow cytometry at 0 h, 24 h, and 48 h, respectively, to test the proportion of residual target cells. The summary of results of 3 independent repeated experiments is shown in FIG. 12. The killing results show that KO-7CAR can mediate specific killing of CD7 expression positive cell lines at E: T ratios of 5: 1 and 1: 1.

### 2. Co-culture of T/CAR-T cells with primary tumor cells from T-ALL patients

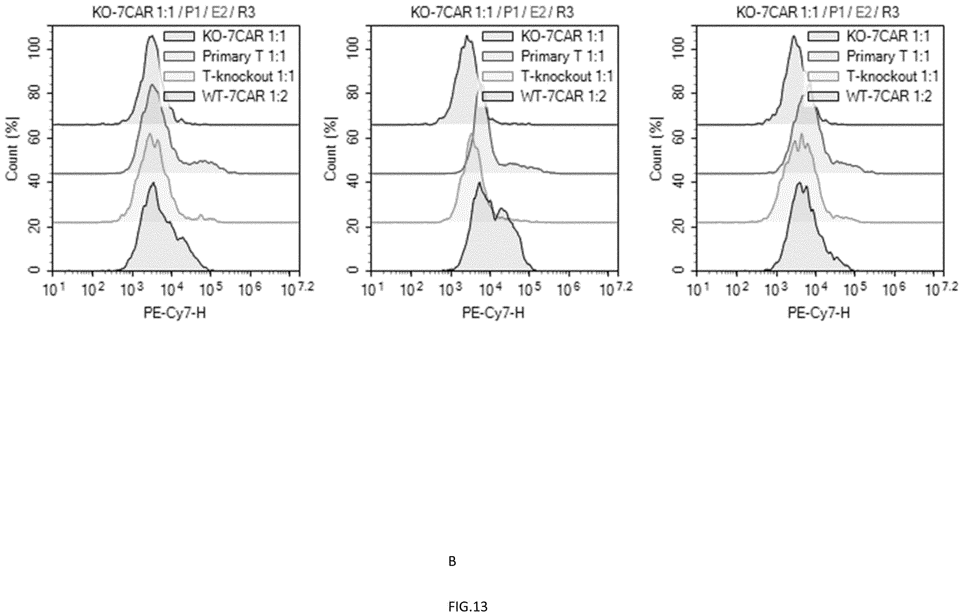

Before co-culture, patients' primary cells (n=3, denoted as P1, P2, P3) were stained with a DiD membrane dye to distinguish T/CAR-T cells from target cells. The patients' primary cells were inoculated into 24-well culture plates (total culture system of 1 ml) at 2×10⁵ cells/well, and 2×10⁵ (E: T = 1: 1), 1×10⁵ (E: T = 1: 2), and 5×10⁴ (E: T = 1: 4) of CAR modified T cells were added respectively. Primary T and T-knockout cells were used as control groups. The cells were co-cultured in an incubator. The proportion of residual target cells was tested by flow cytometry at 0 h, 24 h, and 48 h, respectively. After co-culture for 48 h, the cells were labeled with a PE anti-human CD7 antibody to test the CD7 expression of residual target cells. The results of killing primary cells from 3 patients are shown in FIG. 13. The A results in FIG. 13 show that, compared to primary T and T-knockout, both WT-7CAR and KO-7CAR can mediate killing on patients' primary cells, and the killing effect of KO-7CAR was superior to that of WT-7CAR. The B results in FIG. 13 show that, the patients' primary cells labeled 48 h after co-culture can express the CD7 antigen, while the CD7 antigen cannot be detected in the KO-7CAR co-culture group, indicating that the KO-7CAR can mediate specific killing on tumor cells, and the remaining control groups (primary T, T-knockout, WT-7CAR) had residual CD7 antigen expression positive cells.

### 3) Test on degranulation of T/CAR-T cells:

### 1. Co-culture test on T/CAR-T cells and target cell lines

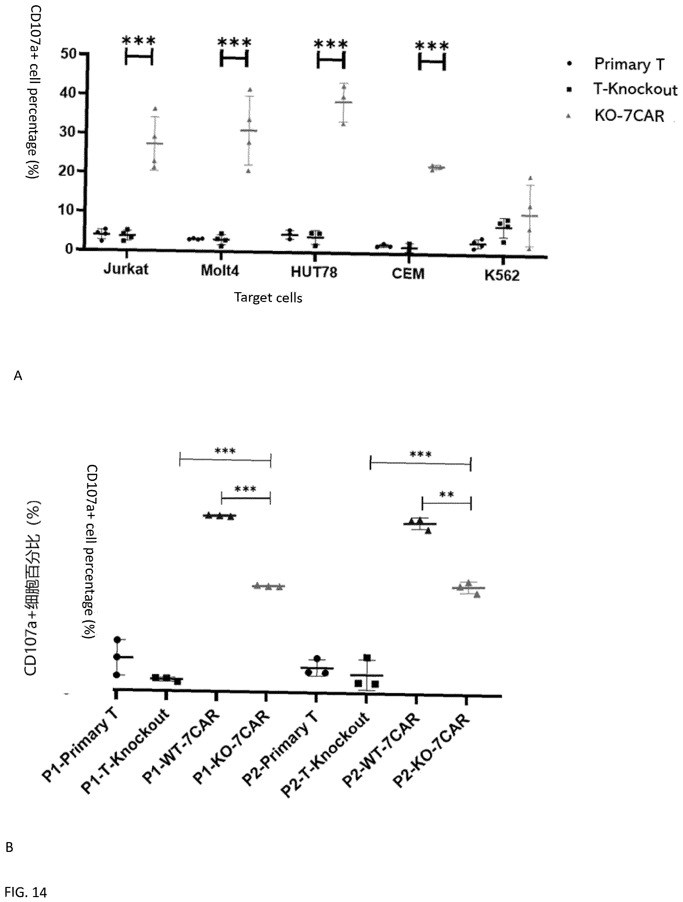

Primary T, T-knockout, and KO-7CAR cells were co-cultured with Jurkat, Molt4, CEM, Hut78, and K562 cell lines in an effector target ratio of 1: 1 on 96-well plates, with a target cell count of 1×10⁵ and a culture system of 200 µl. 1 µl of PE/Cy7 anti-human CD107a antibody and 0.5 µl of 50 U/ml IL-2 were added to the co-culture system. After 1 hour of co-culture, monensin (final concentration 2 µM) was added to the 96-well plate system in dark. After 4 hours, the expression level of CD107a on the surface of T cells was tested with a flow cytometer. 3 repeated wells were set in each group. The results of 3 repeated experiments are as shown in A of FIG. 14. Compared with the primary T and T-knockout groups, when KO-7CAR cells were co-cultured with CD7 positive target cell lines, the CD107a expression was significantly increased, and there was no significant difference in CD107a expression among other three groups of T cells co-cultured with K562 cells.

### 2. Co-culture test on T/CAR-T cells and patients' primary cells

Primary T, T-knockout, WT-7CAR, and KO-7CAR cells were co-cultured with primary cells from 2 patients (the patients' primary cells were pre-stained with a DiD membrane dye) in an effector target ratio of 1: 1 on 96-well plates, with a target cell count of 1×10⁵ and a culture system of 200 µl. 1 µl of PE/Cy7 anti-human CD107a antibody and 0.5 µl of 50 U/ml IL-2 were added to the co-culture system. After 1 hour of co-culture, monensin (final concentration 2 µM) was added to the 96-well plate system in dark. After 4 hours, the expression level of CD107a on the surface of DiD negative cells (i.e., T cells) was tested with a flow cytometer. 3 repeated wells were set in each group. The results of cell degranulation are as shown in B of FIG. 14. Compared with primary T and T-knockout, the CD107a expression of the CD7 CAR-T group was significantly increased, and the degranulation level of WT-7CAR was slightly higher than that of KO-7CAR.

### Example 9: Test on in vivo functions of universal CAR-T cells

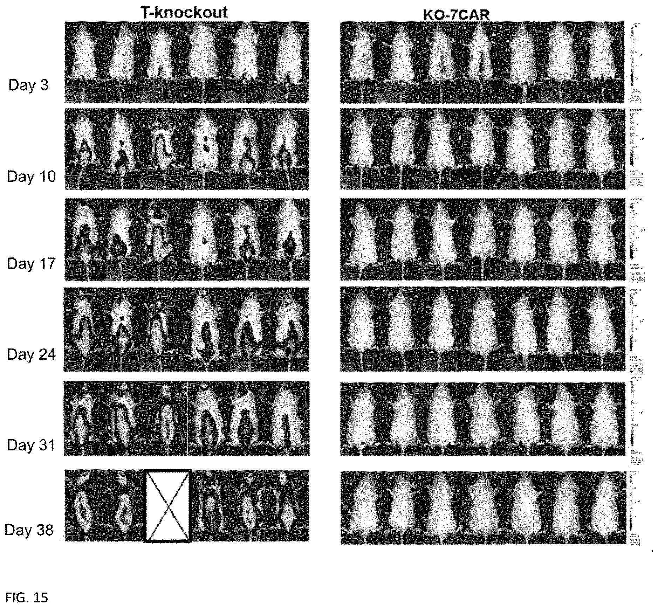

8-10 week old NSG mice were selected, inoculated with 5×10⁵ Jurkat luc (expressing fluorescein) cells through the caudal vein (denoted as day0), and inoculated with 2.5×10⁶T-knockout cells (control group) or KO-7CAR cells (experimental group) through the caudal vein on day4 and day8 respectively. The tumor load in the control group and experimental group mice was monitored weekly using small animal living imaging technology to reflect the tumor clearance function of universal CAR-T cells in vivo. The small animal living imaging results are shown in FIG. 15.

### Example 10: Cross reaction of antibody or antigen-binding moiety with human CD7 negative cells

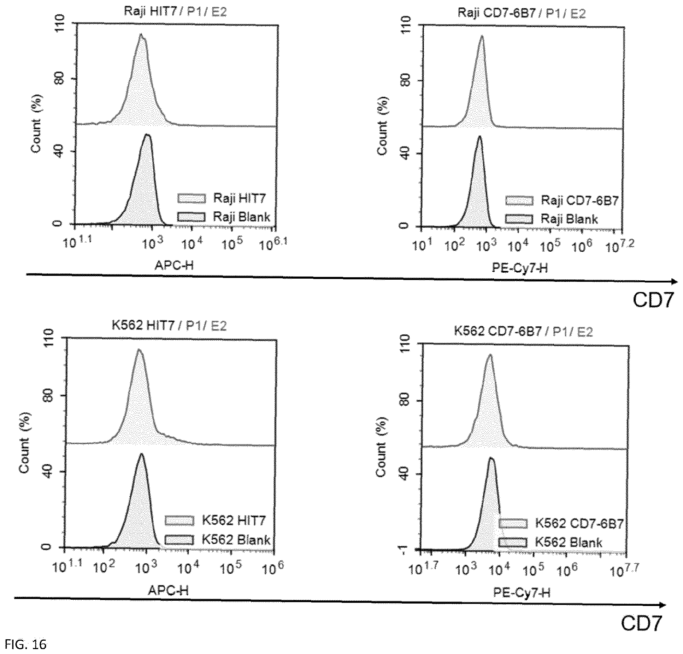

The monoclonal antibody HIT7 in Example 1 was incubated at a concentration of 0.15 µg/100 µl with 2×10⁵ Raji cells (Burkitt lymphoma cell line, CD7 negative) and 2×10⁵ K562 cells (chronic myeloid leukemia cell line, CD7 negative) respectively, and a CD7 commercialized antibody (clone number: CD7-6B7) was used as a control. The cells were incubated at 4°C in dark for 30 minutes, washed twice with PBS, resuspended by 100 µl, and loaded for FACS test. The results are as shown in FIG. 16. The results show that the monoclonal antibody or scFv of the present invention and the control commercialized antibody CD7-6B7 cannot bind to Raji cells and K562 cells, and there was no cross reaction.

### Example 11: Specifically binding of antibody or antigen-binding moiety to human CD7 positive cells (Jurkat, Molt4, CCRF CEM, Hut78 cells)

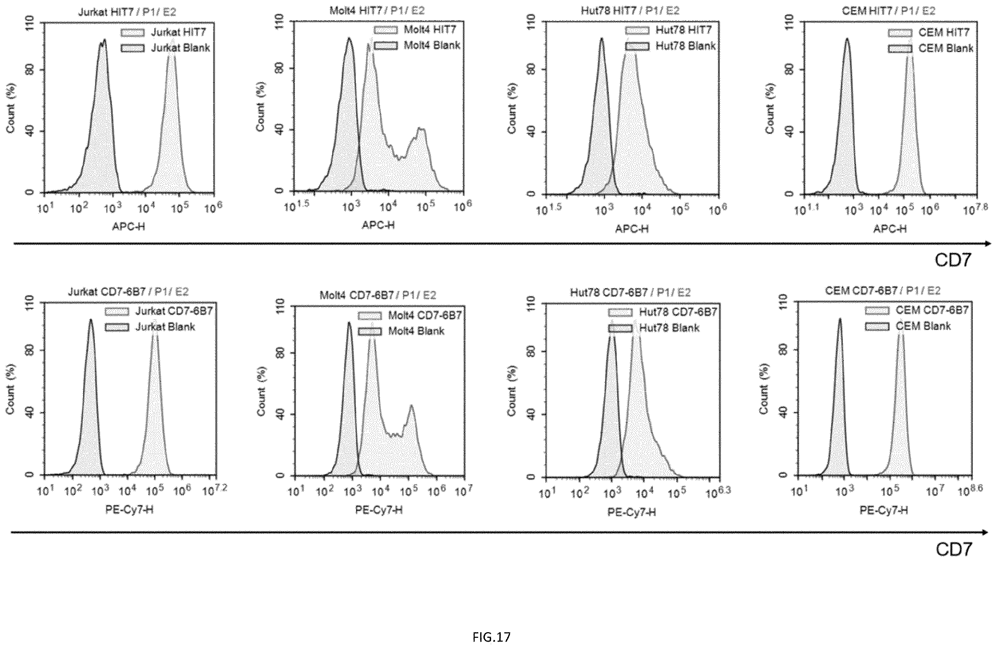

Binding of the monoclonal antibody HIT7 in Example 1 and the commercialized CD7-6B7 antibody (Biolegend, PE/Cyanine7 anti-human CD7 antibody, clone number CD7-6B7) to CD7 on the surface of Jurkat, Molt4, CCRF-CEM, and Hut78 cells that highly expressed human CD7 was tested by FACS: The antibody was incubated at a concentration of 0.15 µg/100 µl with 2×10⁵ Jurkat, Molt4, CEM, and Hut78 cells respectively at 4°C in dark for 30 minutes, washed twice with PBS, resuspended by 100 µl, and loaded for FACS test. The results are as shown in FIG. 17. The results indicate that the monoclonal antibody or scFv of the present invention can effectively bind to Jurkat, Molt4, CEM, and Hut78 cells, with strong affinity and no difference in binding capability from the commercialized antibody CD7-6B7.

### Example 12: Competitive experiment on antibody or antigen-binding moiety and commercialized antibody CD7-6B7 tested by FACS

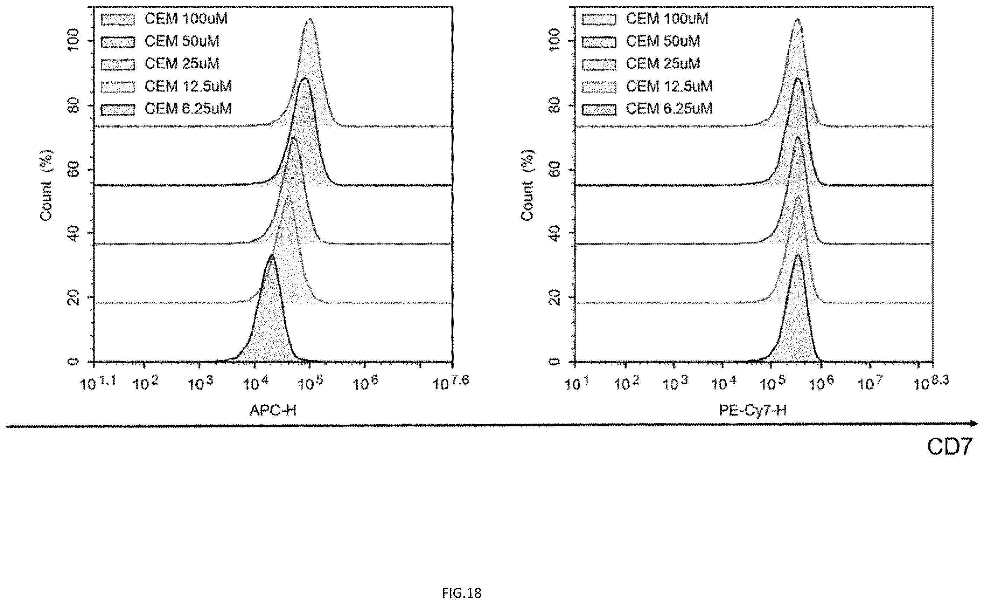

The monoclonal antibody HIT7 in Example 1 was diluted with PBS to 100 µM, 50 µM, 25 µM, 12.5 µM, and 6.25 µM. Meanwhile, 2.5 µl of commercialized antibody was added to each tube. After well mixed, 2×10⁵ CEM cells were incubated at 4°C in dark for 30 minutes, washed twice with PBS, resuspended by 100 µl, and loaded for FACS test. The results are as shown in FIG. 18. The results indicate that the monoclonal antibody or scFv of the present invention cannot compete with the commercialized antibody CD7-6B7 (Biolegend, PE/Cyanine7 anti-human CD7 antibody, clone number CD7-6B7) for binding to the CD7 antigen, showing that the antigen binding epitopes of the two antibodies are different.

Described above are merely preferred implementations of the present invention. It should be pointed out that many improvements and modifications can be made for those of ordinary skill in the art without departing from the principle of the present invention, and these improvements and modifications shall fall into the scope of protection of the present invention.

## Claims

1. An antibody specifically binding to CD7, **characterized in that** the antibody comprises CDR1, CDR2, and CDR3 sequences of a light chain variable region as shown in SEQID No. 1-3 and CDR1, CDR2, and CDR3 sequences of a heavy chain variable region as shown in SEQID No. 4-6, or sequences having more than 80% of homology to the sequences.

2. An antigen-binding moiety specifically binding to CD7, **characterized in that** the antigen-binding moiety comprises CDR1, CDR2, and CDR3 sequences of a light chain variable region as shown in SEQID No. 1-3 and CDR1, CDR2, and CDR3 sequences of a heavy chain variable region as shown in SEQID No. 4-6, or sequences having more than 80% of homology to the sequences.

3. The antigen-binding moiety according to claim 2, **characterized in that** the antigen-binding moiety is Fab, Fab', F(ab')₂, Fd, Fv, scFv, or a single domain antibody (dAB).

4. The antigen-binding moiety according to claim 3, **characterized in that** the antigen-binding moiety is scFv, and the scFv is in a monovalent, divalent, or trivalent form;
wherein the amino acid sequence of the light chain variable region of the monovalent scFv is as shown in SEQID No. 7, and the amino acid sequence of the heavy chain variable region of the monovalent scFv is as shown in SEQID No. 8, or a sequence having more than 80% of homology to the sequence; and
preferably, the amino acid sequence of the monovalent scFv is as shown in SEQID No. 9, or a sequence having more than 80% of homology to the sequence.

5. A polynucleotide, **characterized in that** the polynucleotide encodes the antibody according to claim 1 or the antigen-binding moiety according to claim 2.

6. A vector, **characterized in that** the vector comprises a sequence of the polynucleotide according to claim 5.

7. An isolated cell, **characterized in that** the cell comprises the antibody according to claim 1, the antigen-binding moiety according to claim 2, the polynucleotide according to claim 5, or the vector according to claim 6; and
the cell is selected from a SP2/0, YB2/0, IR983F, PERC6, or CHO cell line, or an insect cell line.

8. A chimeric antigen receptor, comprising an extracellular region, a transmembrane region, and an intracellular region, the intracellular region comprising an intracellular signal transduction region, **characterized in that** the extracellular region of the chimeric antigen receptor comprises a CD7-binding domain, and the CD7-binding domain comprises the antigen-binding moiety according to claim 2.

9. The chimeric antigen receptor according to claim 8, **characterized in that** the extracellular region further comprises a signal peptide constructed at an amino terminal of the chimeric antigen receptor or an amino acid sequence having more than 90% of homology to the signal peptide;
preferably, the signal peptide is of a signal peptide sequence in CD8α or is GM-CSF; and
more preferably, the signal peptide is a signal peptide as shown in SEQ ID No. 10.

10. The chimeric antigen receptor according to claim 8, **characterized in that** the CD7-binding domain is connected to the transmembrane region through a hinge region;
the hinge region is preferably of a hinge region sequence in CD8α;
the transmembrane region is a transmembrane domain selected from the following proteins or an amino acid sequence having more than 90% of homology to the proteins: α, β, or ζ chain of a T cell receptor, CD2, CD3ε, CD4, CD7, CD8α, CD8β, CD11a, CD11b, CD11c, CD11d, CD18, CD19, CD27, CD28, CD29, CD30, CD40, CD48, CD49a, CD49d, CD49f, CD66a, CD66b, CD66c, CD66d, CD66e, CD69, CD79A, CD79B, CD84, CD96, CD100, CD103, CD134, CD137, CD150, CD158A, CD158B1, CD158B2, CD158C, CD158D, CD158F1, CD158F2, CD158K, CD160, CD162, CD226, CD229, CD244, CD247, CD258, CD268, CD270, CD272, CD276, CD279, CD314, CD319, CD335, CD336, CD337, CD352, CD353, CD355, CD357, LFA-1, NKG2C, DAP-10, ICAM-1, NKp80, IL-2R beta, IL-2Rgamma, IL-7R alpha, LFA-1, SLAMF9, LAT, GADS, SLP-76, PAG1/CBP, CD83 ligand, Fc gamma receptor, integrin, activating NK cell receptor, or Toll ligand receptor, or a combination thereof; and
preferably, the transmembrane region is of a transmembrane region sequence in CD8α.

11. The chimeric antigen receptor according to claim 8, **characterized in that** the intracellular region further comprises a co-stimulatory factor;
preferably, the co-stimulatory factor is one or more of functional signal domains obtained from the following proteins or amino acid sequences having more than 90% of homology to the proteins: integrin, BTLA, Toll ligand receptor, OX40, CD2, CD7, CD27, CD28, CD30, CD40, CDS, ICAM-1, LFA-1, 4-1BB, B7-H3, CD278, GITR, BAFFR, LIGHT, HVEM, KIRDS2, SLAMF7, NKp80, NKp44, NKp30, NKp46, CD19, CD4, CD8α, CD8β, IL2Rβ, IL2Rγ, IL7Rα, ITGA4, VLA1, CD49α, IA4, CD49D, ITGA6, VLA6, CD49f, ITGAD, CD11d, ITGAE, CD103, ITGAL, CD11α, ITGAM, CD11b, ITGAX, CD11c, CD29, ITGB1, ITGB2, CD18, ITGB7, NKG2D, NKG2C, TNFR2, CD226, CD84, CD96, CEACAM1, CRTAM, CD229, CD160, PSGL1, CD100, CD69, SLAMF6, SLAM, BLAME, CD162, LTBR, LAT, GADS, or SLP-76; and
more preferably, the co-stimulatory factor is CD28 or 4-1BB, or an amino acid sequence having more than 90% of homology to the CD28 or 4-1BB.

12. The chimeric antigen receptor according to claim 8, **characterized in that** the intracellular signal transduction region is selected from the following proteins or an amino acid sequence having more than 90% of homology to the proteins: 4-1BB, B7-H3, BAFFR, BLAME, BTLA, CD100, CD103, CD160, CD18, CD19, CD19a, CD2, CD247, CD27, CD276, CD28, CD29, CD3ζ, CD30, CD4, CD40, CD49a, CD49D, CD49f, CD69, CD7, CD84, CD8alpha, CD8beta, CD96, CDS, CEACAM1, CRTAM, DAP-10, DNAM1, Fc gamma receptor, GADS, GITR, HVEM, IA4, ICAM-1, ICAM-1, Ig alpha, IL2R beta, IL2R gamma, IL7R alpha, integrin, ITGA4, ITGA4, ITGA6, ITGAD, ITGAE, ITGAL, ITGAM, ITGAX, ITGB2, ITGB7, KIRDS2, LAT, LFA-1, LFA-1, LIGHT, LIGHT, LTBR, Ly9, NKG2C, NKG2D, NKp30, NKp44, NKp46, NKp80, OX-40, PAG/Cbp, PD-1, PSGL1, SELPLG, SLAMF4, SLAMF6, SLAMF7, SLP-76, TNFR2, Toll ligand receptor, TRANCE/RANKL, VLA1, or VLA-6, or a combination thereof; and
preferably, the intracellular signal transduction region is CD3ζ.

13. The chimeric antigen receptor according to claim 8, **characterized in that** the amino acid sequence of the chimeric antigen receptor is as shown in SEQID No. 11, or an amino acid sequence having more than 90% of homology to the amino acid sequence.

14. A polynucleotide, **characterized in that** the polynucleotide encodes the chimeric antigen receptor according to claim 8; and
preferably, the sequence of the polynucleotide is as shown in SEQID No. 12.

15. An isolated cell, **characterized in that** the cell comprises the antigen-binding moiety according to claim 2, the chimeric antigen receptor according to claim 8, or a sequence of the polynucleotide according to claim 5 or 14;
the cell is a T cell or an NK cell; and
preferably, the T cell is either an autologous T cell or a allogeneic T cell.

16. The cell according to claim 15, **characterized in that** the cell is a allogeneic T cell, and the T cell is a universal chimeric antigen receptor T cell (CAR-T);
preferably, the T cell lacks a CD7 gene sequence; and
more preferably, the T cell also lacks a TRAC gene sequence.

17. A use of the antibody according to claim 1, the antigen-binding moiety according to claim 2, the polynucleotide according to claim 5 or 14, the vector according to claim 6, the isolated cell according to claim 7, 15, or 16, or the chimeric antigen receptor according to claim 8 in preparing a drug for treating a tumor;
preferably, the tumor is a hematologic tumor;
more preferably, the tumor is a T cell hematologic tumor;
further preferably, the tumor is CD7 antigen positive acute T lymphocytic leukemia or lymphoma; and
further, the tumor is refractory or relapsed CD7 antigen positive acute T lymphocytic leukemia or lymphoma.

18. A pharmaceutical composition, **characterized in that** the pharmaceutical composition comprises the antibody according to claim 1, the antigen-binding moiety according to claim 2, the polynucleotide according to claim 5 or 14, the vector according to claim 6, the isolated cell according to claim 7, 15, or 16, or the chimeric antigen receptor according to claim 8, and a pharmaceutically acceptable vector; and
preferably, the pharmaceutical composition further comprises anti-CD5 CAR-T cells.
